# EUROPEAN PATENT APPLICATION

(11) **EP 4 467 978 A1**
(43) Date of publication of application: **27.11.2024**
(21) Application number: 23175662.8
(22) Date of filing: 26.05.2023
(51) Int. Cl.: G01N 33/50

(54) **PHOSPHORYLATED TRISTETRAPROLIN AS A BIOMARKER AND THERAPEUTIC TARGET FOR ANTI-AGING DRUGS**

(71) Applicant: King Faisal Specialist Hospital & Research Centre, Riyadh, 11211 (SA)
(72) Inventor: KHALID S., Abu Khabar, Riyadh 11211 (SA)
(74) Representative: Engelhard, Markus

(57) **Abstract**

The present invention relates to a method of identifying an anti-aging compound. The present invention further relates to a method of preventing or treating aging in a patient and to a compound for use in a method of preventing or treating aging in a patient. Furthermore, the present invention relates to a method of determining if a patient is likely to respond to a treatment with an anti-aging compound.

## Description

### FIELD OF THE INVENTION

The present invention relates to a method of identifying an anti-aging compound. The present invention further relates to a method of preventing or treating aging in a patient and a compound for use in a method of preventing or treating aging in a patient. Furthermore, the present invention relates to a method of determining if a patient is likely to respond to a treatment with an anti-aging compound.

### BACKGROUND OF THE INVENTION

Aging is a physiological process mediated by numerous biological and genetic pathways which are a driving force for age-related diseases. Aging may involve a progressive decline of physiological function, which eventually may lead to age-related diseases, such as cardiovascular diseases, arthritis, and neurodegenerative diseases. Age-related diseases may be associated with an economic and/or psychological burden for the patient and his/her family. Aging typically involves cellular senescence, e.g. an accumulation of senescent cells in the tissue. Accumulation of senescent cells may affect tissue regeneration and may result in the secretion of a large number of inflammatory factors, e.g. in the presence of a senescence-associated secretory phenotype (SASP). The secretion of various inflammatory factors may have negative effects on the surrounding tissue.

Treatment strategies for preventing or treating aging and age-related diseases have been studied, such as strategies involving diet, exercise, and drugs. Due to occurrence of side effects associated with various drugs, it is important to assess, prior to an administration of a drug, whether a treatment with a certain drug is likely to be successful in a patient. Personalized medicine allows for customizing the specific treatment to a patient's needs, i.e. the patient's genetic and phenotypical features, and thus allows for a targeted treatment of aging in said patient. There remains the need for suitable biomarkers and methods for assessing whether a patient is likely to respond to an anti-aging compound. For example, there is the need for biomarkers and methods that are capable of indicating whether an anti-aging compound effectively evokes a therapeutic effect.

Furthermore, there remains the need for efficient methods of preventing or treating aging, such as methods of preventing or treating cellular senescence, and for compounds for use in such methods. There is also the need for methods of preventing or treating aging in patients with high specificity and for compounds for use in such methods. Moreover, there is the need for methods of identifying anti-aging compounds, such as anti-aging compounds with high specificity, for example compounds which may be used in methods of preventing or treating aging.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a method of identifying an anti-aging compound comprising, in any order, the following steps:
a) providing a sample comprising senescent cells, optionally a tissue sample,
b) optionally, determining a level of phosphorylated TTP in said sample,
c) providing one or more compound(s) to be tested,
d) treating said sample with said one or more compound(s),
e) determining whether said one or more compound(s) reduce(s) the levels of phosphorylated TTP in said treated sample compared to a control,
wherein a reduction in the level of phosphorylated TTP indicates that said one or more compound(s) is/are an anti-aging compound.

In one embodiment, said control in step e) is a level of phosphorylated TTP determined in step b), and/or is a reference value, and/or is a level of phosphorylated TTP determined in a reference sample.

In one embodiment, said reduction is a reduction by at least 15 %, preferably by at least 20 %, more preferably by at least 25 %.

In one embodiment, said level of phosphorylated TTP is determined using an antibody or antigen-binding fragment thereof targeting phosphorylated TTP.

In one embodiment, the one or more compound(s) is/are selected from small molecule inhibitors, antibodies, and antigen-binding fragments thereof; wherein, preferably, the one or more compound(s) is/are selected from protein kinase inhibitors.

In one embodiment, the anti-aging compound is a senolytic compound, a senomorphic compound, and/or a senotherapeutic compound.

In one embodiment, the method further comprises a step of determining whether said one or more compound(s) reduce(s) a senescence-associated secretory phenotype, a level of at least one senescence-associated secretory phenotype protein, and/or a gene expression of at least one senescence-associated secretory phenotype gene, in said treated sample compared to said control;
wherein, optionally,
said determining whether said one or more compound(s) reduce(s) a senescence-associated secretory phenotype (SASP) comprises a SA-β-gal staining and/or measuring a level of a SASP marker, optionally measuring p53, p21, and/or p16,
said determining whether said one or more compound(s) reduce(s) a level of at least one senescence-associated secretory phenotype protein comprises determining protein level(s) of one or more SASP markers, optionally determining p53, p21, and/or p16, e.g. using ELISA, Western blotting, and/or a functional assay such as a protease activity assay, and/or
said determining whether said one or more compound(s) reduce(s) a gene expression of at least one senescence-associated secretory phenotype gene comprises determining gene expression of one or more SASP markers, optionally determining *p53*, *p21*, and/or *p16,* e.g. using RT-PCR and/or mRNA half-life analysis,
wherein, preferably, said SASP marker is selected from IL-1, IL-6, IL-8, IL-5, MIP-1α, and IFN-γ.

In one embodiment, said method further comprises determining whether said one or more compound(s) reduce(s) a level of AU-rich mRNA, optionally a level of AU-rich mRNA of a senescence-associated secretory phenotype gene, in said treated sample compared to said control.

In one embodiment, said step a) comprises providing a sample comprising senescent cells, optionally a tissue sample, of a patient.

In one embodiment, said step a) comprises providing a sample comprising cells and inducing senescence in said cells using a senescence inducing agent, preferably a lipopolysaccharide, to provide a sample comprising senescent cells.

In one embodiment, said sample comprises immune cells, preferably monocytes and/or macrophages; wherein, optionally, said sample comprises THP-1 cells.

In a further aspect, the present invention relates to a method of preventing or treating aging in a patient, preferably a patient having senescent cells or being at risk of acquiring senescent cells, wherein said senescent cells are characterized by an increased level of phosphorylated TTP in senescent cells compared to non-senescent cells;
wherein said method comprises administering a therapeutically effective amount of a compound identified using a method of identifying an anti-aging compound, as defined herein, to a patient in need thereof, and/or
wherein said method comprises administering a therapeutically effective amount of a compound selected from small molecule inhibitors, antibodies, and antigen-binding fragments thereof, preferably selected from protein kinase inhibitors, to a patient in need thereof.

In one embodiment, said method comprises the steps of:
i) providing a sample comprising cells, optionally a tissue sample, of said patient,
ii) determining the level of phosphorylated TTP in said sample,
iii) administering a therapeutically effective amount of said compound(s) to said patient, if there is an increased level of phosphorylated TTP in the sample compared to a control; wherein, preferably, said control is a reference value and/or is a level of phosphorylated TTP determined in a reference sample.

In one embodiment, said compound is selected from acetyl digitoxin, digitoxin, ergotamine, bromocriptine, dihydroergotoxine, dihydroergotamine, ergoloid mesylate, conivaptan, teniposide, tubocurarin, differin, dutasteride, itraconazole, paclitaxel, lurasidone, novobiocin, praziquantel, loperamide, pranlukast, fluspirilene, antrafenine, desloratadin, dihydroergotamine, ergotamine, glimepiride, glipizide, darifenacin, tadalafil, conivaptan, danazol, dutasteride, tasosartan, metocurine, nilotinib, telithromycin, irinotecan, 5-bromo-2-chloro-N-[3-methyl-4-(2-oxo-2H-chromen-3-yl)phenyl]benzamide, maraviroc, and saquinavir;
wherein, preferably, said compound is selected from glipizide, loperamide, praziquantel, glimepiride, fluspirilene, desloratadin, ergotamine, and teniposide.

In one embodiment, said preventing or treating aging comprises preventing or treating immunosenescence and/or inflammaging.

In one embodiment, said preventing or treating aging comprises preventing or treating an aging-associated disease, preferably selected from cardiovascular diseases such as atherosclerosis and artery narrowing e.g. artery stenosis, type-II diabetes, arthritis, Alzheimer's disease, Parkinson's disease, chronic obstructive pulmonary disease, cancer, and stroke.

In one embodiment, said sample, said determining the level of phosphorylated TTP, and said compound are as defined above.

In a further aspect, the present invention relates to a compound for use in a method of preventing or treating aging in a patient, preferably a patient having senescent cells or being at risk of acquiring senescent cells, wherein said senescent cells are characterized by an increased level of phosphorylated TTP in senescent cells compared to non-senescent cells; wherein said compound is a compound identified using a method of identifying an anti-aging compound, as defined herein, and/or
wherein said compound is selected from small molecule inhibitors, antibodies, and antigen-binding fragments thereof, preferably is selected from protein kinase inhibitors.

In one embodiment, said method of preventing or treating aging in a patient comprises the steps of:
i) providing a sample comprising cells, optionally a tissue sample, of said patient,
ii) determining the level of phosphorylated TTP in said sample,
iii) administering a therapeutically effective amount of said compound(s) to said patient, if there is an increased level of phosphorylated TTP in the sample compared to a control; wherein, preferably, said control is a reference value and/or is a level of phosphorylated TTP determined in a reference sample.

In one embodiment, said compound is selected from acetyldigitoxin, digitoxin, ergotamine, bromocriptine, dihydroergotoxine, dihydroergotamine, ergoloid mesylate, conivaptan, teniposide, tubocurarin, differin, dutasteride, itraconazole, paclitaxel, lurasidone, novobiocin, praziquantel, loperamide, pranlukast, fluspirilene, antrafenine, desloratadin, dihydroergotamine, ergotamine, glimepiride, glipizide, darifenacin, tadalafil, conivaptan, danazol, dutasteride, tasosartan, metocurine, nilotinib, telithromycin, irinotecan, 5-bromo-2-chloro-N-[3-methyl-4-(2-oxo-2H-chromen-3-yl)phenyl]benzamide, maraviroc, and saquinavir;
wherein, preferably, said compound is selected from glipizide, loperamide, praziquantel, glimepiride, fluspirilene, desloratadin, ergotamine, and teniposide.

In one embodiment, said preventing or treating aging comprises preventing or treating immunosenescence and/or inflammaging; and/or
said preventing or treating aging comprises preventing or treating an aging-associated disease, preferably selected from cardiovascular diseases such as atherosclerosis and artery narrowing e.g. artery stenosis, type-II diabetes, arthritis, Alzheimer's disease, Parkinson's disease, chronic obstructive pulmonary disease, cancer, and stroke.

In one embodiment, said sample, said determining the level of phosphorylated TTP, and said compound are as defined above.

In a further aspect, the present invention relates to a method of determining if a patient is likely to respond to a treatment with an anti-aging compound, preferably selected from small molecule inhibitors, antibodies, and antigen-binding fragments thereof, more preferably selected from protein kinase inhibitors, wherein the method comprises the following steps:
i) providing a sample of a patient, wherein said sample comprises senescent cells,
ii) determining a level of phosphorylated tristetraprolin (TTP) in said sample, and
iii) comparing the level of phosphorylated TTP determined in step ii) to a control, wherein said control is preferably a reference value and/or a reference sample,

wherein an increased level of phosphorylated TTP in said sample compared to said control indicates that said patient is likely to respond to a treatment using an anti-aging compound;
wherein, optionally, said method further comprises
   providing a sample of said patient, wherein said sample comprises senescent cells, and treating said sample with one or more anti-aging compound(s),
   determining a level of phosphorylated TTP in said treated sample, and,
   comparing the level of phosphorylated TTP determined in said treated sample to the level of phosphorylated TTP determined in step ii),
wherein a decreased level of phosphorylated TTP in said treated sample compared to the level of phosphorylated TTP determined in step ii) indicates that said patient is likely to respond to a treatment with said one or more anti-aging compound(s).

In one embodiment, said determining of a level of phosphorylated TTP is performed using an antibody or antigen-binding fragment thereof targeting phosphorylated TTP and/or TTP.

In one embodiment, said sample, said determining the level of phosphorylated TTP, and said compound are as defined above.

In a further aspect, the present invention relates to the use of a compound for the manufacture of a medicament for preventing or treating aging in a patient,
wherein said compound is a compound identified using a method of identifying an anti-aging compound, as defined herein, and/or
wherein said compound is selected from small molecule inhibitors, antibodies, and antigen-binding fragments thereof, preferably is selected from protein kinase inhibitors.

In one embodiment, said preventing or treating aging comprises preventing or treating immunosenescence and/or inflammaging; and/or
said preventing or treating aging comprises preventing or treating an aging-associated disease, preferably selected from cardiovascular diseases such as atherosclerosis and artery narrowing e.g. artery stenosis, type-II diabetes, arthritis, Alzheimer's disease, Parkinson's disease, chronic obstructive pulmonary disease, cancer, and stroke.

In one embodiment, said preventing or treating aging in a patient and said compound are as defined above.

### DETAILED DESCRIPTION

The present invention aims at, for example, providing a method for identifying effective anti-aging compounds. Furthermore, the present invention aims at providing a biomarker for determining whether a patient is likely to respond to an (anti-)aging treatment and/or for selecting an appropriate drug for a patient. The present invention further aims at providing an effective method of preventing or treating aging, and a compound for use in such a method.

The inventors herein describe that TTP phosphorylation is a useful (anti-)aging biomarker. The analysis of TTP phosphorylation may be used to select anti-aging compounds for the treatment of aging, particularly by monitoring whether an anti-aging compound reduces the level of phosphorylated TTP. The herein described biomarker, particularly phosphorylated TTP, can be used for analyzing whether an anti-aging compound, such as a protein kinase inhibitor, is likely to be effective in the treatment of aging in a patient. Particularly, if levels of phosphorylated TTP are increased in a patient compared to a control, the anti-aging compound is likely to be effective in the treatment of said patient. Furthermore, said biomarker is an efficient tool for selecting the anti-aging compound (from several compound candidates) which is likely to be most effective in a patient, namely by analyzing the response of a sample of a patient to multiple anti-aging compounds and choosing the anti-aging compound which is most effective. Advantageously, using phosphorylated TTP as a biomarker allows for predicting and/or determining the effectiveness of an anti-aging treatment. Advantageously, by determining the level of phosphorylated TTP in a sample of a patient, and optionally by determining the response of said sample to an anti-aging compound (as determined by the level of phosphorylated TTP after treatment with the anti-aging compound), the most successful anti-aging compound for the particular patient may be selected. Accordingly, the biomarker and method(s) are useful tools for personalized anti-aging treatments.

The present invention relates to a method of identifying an anti-aging compound comprising, in any order, the following steps:
a) providing a sample comprising senescent cells, optionally a tissue sample,
b) optionally, determining a level of phosphorylated TTP in said sample,
c) providing one or more compound(s) to be tested,
d) treating said sample with said one or more compound(s),
e) determining whether said one or more compound(s) reduce(s) the levels of phosphorylated TTP in said treated sample compared to a control,
wherein a reduction in the level of phosphorylated TTP indicates that said one or more compound(s) is/are an anti-aging compound. Advantageously, the method of identifying an anti-aging compound according to the invention allows to identify compounds which are effective in treating aging, particularly senescence. Advantageously, the method of the invention uses phosphorylated TTP as a biomarker to identify highly effective anti-aging compounds.

The term "anti-aging compound", as used herein, relates to a compound with anti-aging activity, e.g. a compound which is effective in preventing or treating senescence such as cellular senescence. In one embodiment, the anti-aging compound is a senolytic compound, a senomorphic compound, and/or a senotherapeutic compound. In one embodiment, the anti-aging compound is effective in treating cellular senescence, particularly by reducing a phosphorylation of TTP. In one embodiment, the anti-aging compound is selected from small molecule inhibitors, antibodies, and antigen-binding fragments thereof; wherein, preferably, the anti-aging compound is selected from protein kinase inhibitors. Providing one or more compound(s) to be tested may comprise providing one or more compound(s) suspected of having anti-aging activity and/or providing a compound library comprising a plurality of compounds. In one embodiment, the one or more compound(s) to be tested are selected from small molecule inhibitors, antibodies, and antigen-binding fragments thereof; preferably from protein kinase inhibitors. In one embodiment, the expression "to be tested" relates to being tested with respect to anti-aging activity and/or TTP phosphorylation reducing activity. The anti-aging compound may be selected from acetyldigitoxin, digitoxin, ergotamine, bromocriptine, dihydroergotoxine, dihydroergotamine, ergoloid mesylate, conivaptan, teniposide, tubocurarin, differin, dutasteride, itraconazole, paclitaxel, lurasidone, novobiocin, praziquantel, loperamide, pranlukast, fluspirilene, antrafenine, desloratadin, dihydroergotamine, ergotamine, glimepiride, glipizide, darifenacin, tadalafil, conivaptan, danazol, dutasteride, tasosartan, metocurine, nilotinib, telithromycin, irinotecan, 5-bromo-2-chloro-N-[3-methyl-4-(2-oxo-2H-chromen-3-yl)phenyl]benzamide, maraviroc, and saquinavir; wherein, preferably, the anti-aging compound is selected from glipizide, loperamide, praziquantel, glimepiride, fluspirilene, desloratadin, ergotamine, and teniposide. For example, the anti-aging compound may be selected from small molecule inhibitors, such as selected from acetyldigitoxin, digitoxin, ergotamine, bromocriptine, dihydroergotoxine, dihydroergotamine, ergoloid mesylate, conivaptan, teniposide, tubocurarin, differin, dutasteride, itraconazole, paclitaxel, lurasidone, novobiocin, praziquantel, loperamide, pranlukast, fluspirilene, antrafenine, desloratadin, dihydroergotamine, ergotamine, glimepiride, glipizide, darifenacin, tadalafil, conivaptan, danazol, dutasteride, tasosartan, metocurine, nilotinib, telithromycin, irinotecan, 5-bromo-2-chloro-N-[3-methyl-4-(2-oxo-2H-chromen-3-yl)phenyl]benzamide, maraviroc, and saquinavir; wherein, preferably, the anti-aging compound is selected from glipizide, loperamide, praziquantel, glimepiride, fluspirilene, desloratadin, ergotamine, and teniposide. In one embodiment, the anti-aging compound is or is not selected from dasatinib, quercetin, ABT-737, ABT-263 (navitoclax), A-1331852, A-1155463, 17-DMAG (alvespimycin), geldanamycin, 17-AAG (tanespimycin), ganetespib, FOXO4-DRI, UBX0101, RG7112 (RO5045337), P5091, P22077, fisetin, curcumin, o-vanillin (curcumin metabolite), EF-24 (curcumin analogue), piperlongumine and its analogues (compounds 47-49), GL-V9, proscillaridin A, ouabain, ouabagenin, digoxin, bufalin, k-stropanthin, strophanthidin, SSK1, pro-drug A (JHB75B), nav-gal, 5FURGa, PZ15227, ARV825, fenofibrate, azithromycin, roxithromycin, tamatinib, mitotam, panobinostat, AT-406, rapamycin, metformin, resveratrol, stacs, aspirin, SR12343, SB203580, UR13756, BIRB796, MK2.III, PF-3644022, ruxolitinib, KU-55933, KU-60019, atorvastatin, pravastatin, pitavastatin, simvastatin, apigenin, kaempferol, quercetin, quercetin caprylate, epigallocatechin gallate, genistein, oleuropein aglycone, and hydroxytyrosol.

The term "small molecule inhibitor" relates to a small molecule compound, preferably to a small molecule which inhibits a signaling pathway in a patient's body, preferably a senescence-related signaling pathway, more preferably a signaling pathway related to an aging-associated disease. The term "antigen-binding fragment thereof, as used herein, relates to a peptide that specifically binds to an antigen. In one embodiment, an antigen-binding fragment is based on an immunoglobulin, such as a polyclonal or monoclonal antibody, for example a substantially intact antibody, a Fab fragment, a F(ab')2 fragment, a diabody, a single chain Fv fragment, a tetrabody, a triabody, a disulfide bond-stabilized Fv (dsFv), or a heavy chain VHH fragment from camels, or is based on a protein scaffold structure having antigen-binding capacity, such as an anticalin protein, an Affilin, an Affimer, an Affitin, an Alphabody, a nanobody, or a DARPin, preferably comprising antigen-binding determinants, such as a CDR, of an antibody. In one embodiment, the antibody and/or the antigen-binding fragment thereof target(s) phosphorylated TTP or TTP, i.e. specifically binds to phosphorylated TTP or TTP. The term "protein kinase inhibitor", as used herein, refers to an inhibitor that blocks the action of one or more protein kinases, e.g. dasatinib. In one embodiment, said term relates to an inhibitor that attenuates the action of one or more protein kinases. In one embodiment, said protein kinase inhibitor is a serine/threonine protein kinase inhibitor, such as a B-Raf kinase inhibitor or a polo-like kinase inhibitor, or a tyrosine kinase inhibitor, for example a VEGFR2 inhibitor. The term "PLK-1" or "polo-like kinase 1", as used herein, refers to a specific kinase which is a member of the family of polo-like kinases. In one embodiment, a protein kinase inhibitor is preferably an inhibitor of a MAP kinase, such as an inhibitor of MK2 and/or ERK, an inhibitor of AKT, and/or an inhibitor of ERBB2, such as lapitinib. In one embodiment, the term "compound" and/or "anti-aging compound" relates to a protein kinase inhibitor.

The term "sample", as used herein, relates to a specimen, preferably comprising senescent cells. The sample may be a sample of a patient, of a cell line, of a model organism, an artificial sample, and/or any other suitable sample. In one embodiment, a sample, particularly a patient sample, is any of a solid sample, such as a formalin-fixed and/or paraffin-embedded tissue, a fresh tissue, a frozen tissue, and/or a patient-derived xenograft, and a liquid sample, such as a blood sample, blood total cells, circulating cells, extracellular vesicles, exosomes, lymph fluid, saliva, body fluid, and/or tissue fluid. In a preferred embodiment, the sample comprises senescent cells. In one embodiment, the sample is obtained from a patient, preferably noninvasively obtained from a patient. Typically, a control sample or control value is used to estimate the relative phosphorylation levels of TTP in a senescent cell, organ, or tissue compared to a non-senescent cell, organ, or tissue.

The term "senescent cell", as used herein, refers to a cell that has stopped dividing but has not died. In one embodiment, a senescent cell is a cell characterized by cellular senescence. In one embodiment, senescent cells are characterized by increased levels of phosphorylated TTP, particularly increased levels of phosphorylated TTP compared to non-senescent cells. Cellular senescence can be initiated by a wide variety of stress inducing factors. These stress factors may include both environmental and internal damaging events, abnormal cellular growth, oxidative stress, and autophagy factors. The term "non-senescent cell", as used herein, refers to a cell which is not affected by cellular senescence. In one embodiment, the terms "senescence" and "aging", as used herein, are used interchangeably. In a preferred embodiment, senescent cells are not cancer cells. In one embodiment, a sample comprising senescent cells does not comprise cancer cells.

In one embodiment, said step a) comprises providing a sample comprising senescent cells, optionally a tissue sample, of a patient. Advantageously, when using a patient sample in step a) of a method of identifying an anti-aging compound, the method allows to identify anti-aging compounds which are particularly suitable for the respective patient. Particularly, by using a patient sample, it can be predicted that the identified anti-aging compound has a therapeutic effect in said patient. In one embodiment, said step a) comprises providing a sample comprising cells, such as non-senescent cells, and inducing senescence in said cells using a senescence inducing agent, preferably a lipopolysaccharide, to provide a sample comprising senescent cells. For example, the sample comprising cells may be a patient sample, a sample of a model organism, or a sample of a cell line. The inventors have found that, advantageously, the method of identifying an anti-aging compound can also be performed with a sample comprising non-senescent cells (for example, if a sample comprising senescent cells is not available) and inducing senescence in said non-senescent cells using a senescence inducing agent. In a preferred embodiment, the senescence inducing agent is a lipopolysaccharide.

In one embodiment, said sample comprises immune cells, preferably monocytes and/or macrophages; wherein, optionally, said sample comprises THP-1 cells. In one embodiment, the immune cells, preferably monocytes and/or macrophages, optionally THP-1 cells, are senescent cells or are induced to become senescent cells by a senescence inducing agent. For example, a sample comprising immune cells may be a sample of a patient, a sample of a model organism, or a sample of a cell line. Advantageously, when using a sample comprising immune cells, anti-aging compounds are identified which are effective in preventing or treating immunosenescence and/or inflammaging.

The term "determining a level of phosphorylated TTP", as used herein, relates assessing the level of phosphorylated TTP comprising any method capable of detecting a phosphorylation status of a protein that is known to a person skilled in the art, such as methods using reactions between an antibody (or antigen-binding fragment) and an antigen, said antigen preferably being phosphorylated TTP, for example western blotting, immunohistochemistry, immunofluorescence, mass spectrometry, flow cytometry, FACS, and ELISA. In one embodiment, said determining comprises detecting the total amount of phosphorylated TTP and/or detecting the fraction of phosphorylated TTP compared to total TTP. In one embodiment, an increased level of phosphorylated TTP relates to an increased total amount of phosphorylated TTP and/or to an increased phosphorylation degree of TTP, wherein an increased phosphorylation degree of TTP means that the ratio of phosphorylated TTP to unphosphorylated TTP is increased. In one embodiment, reducing a level of phosphorylated TTP relates to reducing a total amount of phosphorylated TTP and/or to reducing a phosphorylation degree of TTP, wherein a reduced phosphorylation degree of TTP means that the ratio of phosphorylated TTP to unphosphorylated TTP is reduced. In one embodiment, the level of phosphorylated TTP is determined using an antibody targeting phosphorylated TTP and/or is determined using an antibody targeting TTP. In one embodiment, if an antibody targeting TTP is used to determine the level of phosphorylated TTP, the molecular weight and/or size difference between a phosphorylated TTP and an unphosphorylated TTP is taken into account to determine the level of phosphorylated TTP, wherein phosphorylated TTP is larger than TTP, as observed, for example, with the bands obtained in western blotting. In one embodiment, phosphorylated TTP is detected by anti-phosphorylated TTP using western blotting, immunohistochemistry, immunofluorescence, or any other method capable of detecting phosphorylated TTP known to a person skilled in the art. In one embodiment, determining a level of phosphorylated TTP relates to assessing the protein level of phosphorylated TTP and/or unphosphorylated TTP. In one embodiment, phosphorylated TTP is used as a senescence biomarker, and thus the level of phosphorylated TTP is determined in a method of determining if a patient is likely to respond to a treatment according to the present invention and/or a method of identifying an anti-aging compound. In one embodiment, determining a level of phosphorylated TTP comprises using phosphorylated TTP as a biomarker. In one embodiment, said level of phosphorylated TTP is determined using an antibody or antigen-binding fragment thereof targeting phosphorylated TTP.

The term "treating", as used herein, preferably refers to applying a compound, such as an anti-aging compound, to a target such as a patient and/or a sample, e.g. a sample of a patient. In one embodiment, said treating relates to in *vivo* treating of a patient, and/or to *in vitro* treating of a sample such as a sample of a patient.

A "control", as used herein, relates to a reference value and/or a reference sample which preferably reflect(s) the characteristics of a non-senescent subject, non-senescent tissue, and/or non-senescent cell. In one embodiment, the terms "reference sample" and "control sample" are used interchangeably. A "control sample", as used herein, relates to a sample comprising or consisting of non-senescent cells, e.g. a sample for determining normal expression and/or phosphorylation levels in non-senescent cells. Such a control sample may derive from the patient, wherein said control sample is taken from a non-senescent tissue, wherein said non-senescent tissue may derive from the same organ as the sample comprising senescent cells, but a different site not affected by said senescence, or may derive from a different organ not affected by said senescence. A control sample may also relate to a sample of non-senescent tissue and/or non-senescent sample(s) of a group of subjects. In some embodiments, said control sample(s) may also relate to "control values" which reflect the normal expression and/or phosphorylation levels obtained from analysis of expression and/or phosphorylation in control samples, wherein said control samples derive from non-senescent tissue of the patient, or non-senescent tissue of another individual, or non-senescent tissue of a group of subjects. In one embodiment, said control in step e) of said method of identifying an anti-aging compound is a level of phosphorylated TTP determined in step b) of said method of identifying an anti-aging compound, and/or is a reference value, and/or is a level of phosphorylated TTP determined in a reference sample.

In one embodiment, said reduction is a reduction by at least 15 %, preferably by at least 20 %, more preferably by at least 25 %. The inventors have found that the compound is an effective anti-aging compound if phosphorylated TTP is reduced by at least 15 %, preferably by at least 20 %, more preferably by at least 25 % by said compound. Particularly, compounds are highly efficient anti-aging compounds if phosphorylated TTP is reduced by at least 25 %.

In one embodiment, the method further comprises a step of determining whether said one or more compound(s) reduce(s) a senescence-associated secretory phenotype, a level of at least one senescence-associated secretory phenotype protein, and/or a gene expression of at least one senescence-associated secretory phenotype gene, in said treated sample compared to said control. Advantageously, the determination of a SASP, a SASP protein, and/or SASP gene allows to even further enhance the specificity of the method of identifying an anti-aging compound. The inventors have found that, advantageously, a compound which reduces a SASP, a SASP protein level, and/or SASP gene expression in addition to reducing the level of phosphorylated TTP in a treated sample compared to a control has an even further enhanced anti-aging activity.

The term "senescence-associated secretory phenotype" or "SASP", as used herein, relates to a phenotype associated with senescent cells, preferably senescent cells secreting high levels of inflammatory cytokines, immune modulators, growth factors, and/or proteases. In one embodiment, a senescent cell is characterized by a senescence-associated secretory phenotype, optionally is further characterized by arrested cell growth and resistance to apoptosis. Cells with the SASP may be characterized by being in cell cycle arrest, releasing inflammatory factors, and possessing a particular morphology. The term "senescence-associated secretory phenotype protein", as used herein, relates to a protein associated with the senescence-associated secretory phenotype, e.g. a protein which corresponds to a SASP marker. The term "senescence-associated secretory phenotype gene", as used herein, relates to a gene associated with the senescence-associated secretory phenotype, e.g. a gene which corresponds to a SASP marker. In one embodiment, the SASP marker is selected from ANGPT1, AREG, BMP2, CCL13, CCL16, CCL2, CCL3, CCL3L1, CCL4, CCL7, CCL8, CD55, CSF1, CSF2, CXCL1, CXCL10, CXCL12, CXCL2, CXCL3, CXCL8, EDN1, EGF, EREG, ESM1, FAS, FGF2, FGF7, GDF15, IL-6, IGFBP1, IGFBP7, IFN-γ, IL-8, IL-5, IL-10, IL-13, IL-15, IL-1, IL-1A, IL-1B, IL-2, IL-6ST, ITGA2, JUN, MIP-1α, MMP13, MMP3, NAP1L4, PAPPA, PLAT, PLAU, PLAUR, PTBP1, PTGER2, RPS6KA5, SERPINE1, SPX, TIMP2, TNF, VEGFA, VEGFC, WNT2, CXCL5, TFRC, ADSS, PTX3, and STC1. In a preferred embodiment, said SASP marker is selected from IL-1, IL-6, IL-8, IL-5, MIP-1α, and IFN-γ. For example, marker levels, such as SASP marker levels, may be measured by measuring a protein level of said marker and/or gene expression of said marker. Marker levels and/or other targets, such as p53, p21, and/or p16, may be measured by methods such as ELISA, Western blotting, functional assays such as protease activity assays, RT-PCR and/or mRNA half-life analysis.

In one embodiment, said determining whether said one or more compound(s) reduce(s) a senescence-associated secretory phenotype (SASP) comprises a SA-β-gal staining and/or measuring a level of a SASP marker, optionally measuring p53, p21, and/or p16,
said determining whether said one or more compound(s) reduce(s) a level of at least one senescence-associated secretory phenotype protein comprises determining protein level(s) of one or more SASP markers, optionally determining p53, p21, and/or p16, e.g. using ELISA, Western blotting, and/or a functional assay such as a protease activity assay, and/or
said determining whether said one or more compound(s) reduce(s) a gene expression of at least one senescence-associated secretory phenotype gene comprises determining gene expression of one or more SASP markers, optionally determining *p53*, *p21*, and/or *p16,* e.g. using RT-PCR and/or mRNA half-life analysis,
wherein, preferably, said SASP marker is selected from IL-1, IL-6, IL-8, IL-5, MIP-1α, and IFN-γ. The term "SA-β-gal staining", as used herein, relates to senescence-associated beta-galactosidase staining. The skilled person understands that a SA-β-gal staining may be performed using a commercially available kit. Advantageously, by additionally measuring p53, p21, and/or p16, the reliability of the determination of the senescence-associated secretory phenotype, SASP protein level, or SASP gene expression is even further enhanced.

In one embodiment, said method further comprises determining whether said one or more compound(s) reduce(s) a level of AU-rich mRNA, optionally a level of AU-rich mRNA of a senescence-associated secretory phenotype gene, in said treated sample compared to said control. The term "AU-rich mRNA", as used herein, relates to mRNA comprising one or more, preferably a plurality of adenylate-uridylate-rich elements. The inventors have found that, advantageously, a compound which reduces a level of AU-rich mRNA in addition to reducing the level of phosphorylated TTP in a treated sample compared to a control has an even further enhanced anti-aging activity.

The present invention also relates to a method of preventing or treating aging in a patient, preferably a patient having senescent cells or being at risk of acquiring senescent cells, wherein said senescent cells are characterized by an increased level of phosphorylated TTP in senescent cells compared to non-senescent cells;
wherein said method comprises administering a therapeutically effective amount of a compound identified using a method of identifying an anti-aging compound, as defined herein, to a patient in need thereof, and/or
wherein said method comprises administering a therapeutically effective amount of a compound selected from small molecule inhibitors, antibodies, and antigen-binding fragments thereof, preferably selected from protein kinase inhibitors, to a patient in need thereof. Advantageously, with a method of preventing or treating aging in a patient according to the invention, aging such as cellular senescence can be effectively treated. The embodiments and definitions described above in the context of the method of identifying an anti-aging compound also apply to the method of preventing or treating aging a patient of the invention. In one embodiment, the method of preventing or treating aging in a patient is a method of preventing or treating cellular senescence in said patient.

In one embodiment, the method of preventing or treating aging comprises administering a therapeutically effective amount of a compound identified using a method of identifying an anti-aging compound, wherein step a) of said method of identifying an anti-aging compound comprises providing a sample of the patient to be treated with the method of preventing or treating aging.

In one embodiment, preventing or treating aging comprises or consists of preventing or treating cellular senescence, optionally cellular senescence associated with immunosenescence and/or inflammaging. In one embodiment, the term "aging", as used herein, relates to cellular senescence. In one embodiment, preventing or treating aging comprises preventing or treating cellular senescence in a patient, particularly a patient characterized by having cells with an increased level of phosphorylated TTP. Advantageously, the method of preventing or treating aging of the invention is highly effective in treating aging, for example treating cellular senescence, in a patient characterized by having an increased level of phosphorylated TTP. The term "patient", as used herein, refers to a human or an animal having senescent cells, particularly senescent cells which are characterized by increased levels of phosphorylated TTP in senescent cells compared to non-senescent cells. In one embodiment, the patient is characterized by increased levels of phosphorylated TTP. In one embodiment, the patient is characterized by having senescent cells with increased levels of phosphorylated TTP. The terms "subject" and "individual", as used herein, are used synonymously, and preferably relate to a human or an animal.

The terms "increased TTP phosphorylation", "increased phosphorylation", and "increased level of phosphorylated TTP", as used herein, refer to an elevated phosphorylation level of TTP in a sample, particularly a sample of a patient, compared to the phosphorylation level of TTP in a control, optionally referred to as "normal phosphorylation". In some embodiments, phosphorylation of senescent cells is compared to normal phosphorylation in a control sample, wherein the control sample may derive from non-senescent cells or tissue of the same individual, wherein said non-senescent tissue may derive e.g. from a different site of the same organ as the senescent tissue, or which may derive from a non-senescent individual. In some embodiments, phosphorylation of senescent cells is compared to a mean phosphorylation of a group of non-senescent subjects. An elevated phosphorylation level may also be referred to as "increased phosphorylation level". In one embodiment, an increased phosphorylation is an at least 5 % increased phosphorylation level, preferably at least 15 % increased phosphorylation level in a senescent sample compared to a control. The terms "decreasing phosphorylation" or "reducing phosphorylation", as used herein, relates to decreasing elevated phosphorylation levels of TTP, to normalize said increased phosphorylation to normal phosphorylation, preferably by administering an anti-aging compound such as a protein kinase inhibitor. In one embodiment, said decreasing phosphorylation comprises a decrease by at least 15 %, preferably by at least 20 %, more preferably by at least 25 %. Methods for determining the phosphorylation level of a protein such as TTP are known to a person skilled in the art, and include western blot, ELISA, microarrays, immunohistochemistry, immunofluorescence, and mass spectrometry.

The term "normal phosphorylation" or "normal phosphorylation levels", as used herein, refers to phosphorylation levels in non-senescent cells. In one embodiment, normal phosphorylation relates to phosphorylation levels of TTP in non-senescent cells. In one embodiment, normal phosphorylation levels of TTP are assessed in a non-senescent sample of the same subject from which the sample comprising senescent cells is taken. In one embodiment, normal phosphorylation levels are assessed in a sample from a non-senescent subject. In one embodiment, normal phosphorylation levels are assessed in a population of non-senescent individuals. In one embodiment, the term "non-senescent sample" relates to a sample which does not comprise senescent cells. In one embodiment, the terms "non-senescent subject" and "non-senescent individual" relate to a subject and individual, respectively, which substantially do not comprise senescent cells, tissues, or organs. The terms "normalizing" and "normalizing phosphorylation", as used herein, relate to normalizing or restoring phosphorylation levels of TTP to non-senescent phosphorylation levels, which can be achieved by administering a therapeutically effective amount of an anti-aging compound such as a protein kinase inhibitor to a patient in need thereof having increased TTP phosphorylation levels. In one embodiment, a "normalizing effect" refers to an effect, preferably an effect of an anti-aging compound, which induces a normalization of increased TTP phosphorylation levels in senescent cells towards the TTP phosphorylation levels typically found in non-senescent cells.

The term "administering", as used herein, refers to applying an anti-aging compound, such as a protein kinase inhibitor, to a target, such as a patient and/or a sample of a patient. In one embodiment, administering relates to *in vitro* and/or *in vivo* administration. In one embodiment, administering relates to intravenous, oral, nasal, mucosal, intrabronchial, intrapulmonary, intradermal, subcutaneous, intramuscular, intravascular, intrathecal, intraocular, intraarticular, intranodal, intratumoral, or intrametastatical administration of an anti-aging compound to a patient in need thereof. In one embodiment, administering may also relate to *in vitro* administration, namely to incubating a cell and/or tissue, e.g. a sample obtained from a patient, with an anti-aging compound.

The term "therapeutically effective amount", as used herein, refers to an amount of a drug, such as an anti-aging compound, which is in the range between the dose sufficient to evoke a therapeutic effect and the maximum tolerated dose. In one embodiment, a method of preventing or treating aging according to the present invention comprises administering a therapeutically effective amount of an anti-aging compound to a patient in need thereof having an increased level of phosphorylated TTP compared to a control. In one embodiment, said therapeutically effective amount is in a dose range established for a different method of treatment comprising administering said compound, wherein said different method of treatment is for a disease which is not characterized by increased TTP phosphorylation levels in pathophysiological cells compared to physiological cells.

Personalized medicine has the advantage that methods of prevention or treatment are applied to patients that are highly susceptible to the respective method of prevention of treatment. Advantageously, the method of the invention can be used in the context of personalized medicine, particularly by applying the method to patients that are highly susceptible to preventing or treating aging. For example, the method of preventing or treating aging is applied to highly susceptible patients, if the method comprises the steps of:
i) providing a sample comprising cells, optionally a tissue sample, of said patient,
ii) determining the level of phosphorylated TTP in said sample,
iii) administering a therapeutically effective amount of said compound(s) to said patient, if there is an increased level of phosphorylated TTP in the sample compared to a control; wherein, preferably, said control is a reference value and/or is a level of phosphorylated TTP determined in a reference sample.

Advantageously, phosphorylated TTP can be used as a biomarker to identify patients that are highly susceptible to the method of preventing or treating aging of the invention.

In one embodiment, said preventing or treating aging comprises preventing or treating immunosenescence and/or inflammaging. The term "inflammaging", as used herein, relates to a chronic low-grade inflammation that develops with advanced age, typically in the absence of overt infection. Inflammaging may contribute to clinical manifestations of other age-related pathologies. The term "immunosenescence", as used herein, relates to a deterioration of the immune system, particularly associated with age advancement.

In one embodiment, said preventing or treating aging comprises preventing or treating an aging-associated disease, preferably selected from cardiovascular diseases such as atherosclerosis and artery narrowing e.g. artery stenosis, type-II diabetes, arthritis, Alzheimer's disease, Parkinson's disease, chronic obstructive pulmonary disease, cancer, and stroke. In one embodiment, said aging-associated disease is selected from cardiovascular diseases such as atherosclerosis and artery narrowing e.g. artery stenosis, type-II diabetes, arthritis, Alzheimer's disease, Parkinson's disease, chronic obstructive pulmonary disease, cancer, and stroke. In a preferred embodiment, the aging-associated disease is selected from cardiovascular diseases such as atherosclerosis and artery narrowing e.g. artery stenosis, type-II diabetes, arthritis, Alzheimer's disease, Parkinson's disease, chronic obstructive pulmonary disease, and stroke. In one embodiment, the aging-associated disease is not cancer. In one embodiment, the method of preventing or treating aging does not comprise preventing or treating cancer.

In one embodiment, said method of preventing or treating aging comprises monitoring a treatment response in said patient. The term "monitoring a treatment response", as used herein, relates to evaluating the therapeutic success of a treatment, particularly a treatment with an anti-aging compound. The monitoring of the treatment response comprises providing a sample of a first time point and providing a sample of a second time point, wherein the second time point is later in a period of treatment than the first time point, and comparing the level of phosphorylated TTP determined in the sample of the first time point to the level of phosphorylated TTP determined in the sample of the second time point. In one embodiment, if the level of phosphorylated TTP decreases during the period of treatment, i.e. decreases from a first time point to a second time point, the treatment, e.g. an anti-aging compound administered to the patient, is successful in treating said patient.

The present invention also relates to a compound for use in a method of preventing or treating aging in a patient, preferably a patient having senescent cells or being at risk of acquiring senescent cells, wherein said senescent cells are characterized by an increased level of phosphorylated TTP in senescent cells compared to non-senescent cells;
wherein said compound is a compound identified using a method of identifying an anti-aging compound, as defined herein, and/or
wherein said compound is selected from small molecule inhibitors, antibodies, and antigen-binding fragments thereof, preferably is selected from protein kinase inhibitors. Advantageously, with a compound for use of the invention, aging such as cellular senescence can be effectively treated. The embodiments and definitions described above in the context of the method of identifying an anti-aging compound and/or in the context of a method of preventing or treating aging also apply to the compound for use in a method of preventing or treating aging according to the invention. In a preferred embodiment, the compound for use of the invention is for use in a method of preventing or treating aging of the invention.

In one embodiment, the compound is for use in a method of preventing or treating aging in a patient comprising the steps of:
i) providing a sample comprising cells, optionally a tissue sample, of said patient,
ii) determining the level of phosphorylated TTP in said sample,
iii) administering a therapeutically effective amount of said compound(s) to said patient, if there is an increased level of phosphorylated TTP in the sample compared to a control; wherein, preferably, said control is a reference value and/or is a level of phosphorylated TTP determined in a reference sample.

Advantageously, when the compound is for use in a method of preventing or treating aging comprising steps i)-iii), the compound is applied to patients that are highly susceptible to preventing or treating aging. The inventors have found that, advantageously, phosphorylated TTP can be used as a biomarker for identifying patients highly susceptible to aging prevention or treatment.

In one embodiment, the compound for use is an anti-aging compound as defined above. In one embodiment, the compound for use is a senolytic compound, a senomorphic compound, and/or a senotherapeutic compound. In one embodiment, the anti-aging compound is effective in treating cellular senescence, particularly by reducing a phosphorylation of TTP. In one embodiment, the compound is selected from acetyldigitoxin, digitoxin, ergotamine, bromocriptine, dihydroergotoxine, dihydroergotamine, ergoloid mesylate, conivaptan, teniposide, tubocurarin, differin, dutasteride, itraconazole, paclitaxel, lurasidone, novobiocin, praziquantel, loperamide, pranlukast, fluspirilene, antrafenine, desloratadin, dihydroergotamine, ergotamine, glimepiride, glipizide, darifenacin, tadalafil, conivaptan, danazol, dutasteride, tasosartan, metocurine, nilotinib, telithromycin, irinotecan, 5-bromo-2-chloro-N-[3-methyl-4-(2-oxo-2H-chromen-3-yl)phenyl]benzamide, maraviroc, and saquinavir;
wherein, preferably, said compound is selected from glipizide, loperamide, praziquantel, glimepiride, fluspirilene, desloratadin, ergotamine, and teniposide.

In one embodiment, said compound for use is for use in preventing or treating immunosenescence and/or inflammaging. Advantageously, by administering the compound to the patient, the immune system of the patient is strengthened. In one embodiment, said compound for use is for use in preventing or treating an aging-associated disease, preferably selected from cardiovascular diseases such as atherosclerosis and artery narrowing e.g. artery stenosis, type-II diabetes, arthritis, Alzheimer's disease, Parkinson's disease, chronic obstructive pulmonary disease, cancer, and stroke. In one embodiment, the aging-associated disease is not cancer. In a preferred embodiment, the compound is for use in preventing or treating an aging-associated disease selected from cardiovascular diseases such as atherosclerosis and artery narrowing e.g. artery stenosis, type-II diabetes, arthritis, Alzheimer's disease, Parkinson's disease, chronic obstructive pulmonary disease, and stroke. In one embodiment, the compound for use is for use in a method of preventing or treating aging which does not comprise preventing or treating cancer.

The present invention also relates to a method of determining if a patient is likely to respond to a treatment with an anti-aging compound, preferably selected from small molecule inhibitors, antibodies, and antigen-binding fragments thereof, more preferably selected from protein kinase inhibitors, wherein the method comprises the following steps:
i) providing a sample of a patient, wherein said sample comprises senescent cells,
ii) determining a level of phosphorylated tristetraprolin (TTP) in said sample, and
iii) comparing the level of phosphorylated TTP determined in step ii) to a control, wherein said control is preferably a reference value and/or a reference sample,
wherein an increased level of phosphorylated TTP in said sample compared to said control indicates that said patient is likely to respond to a treatment using an anti-aging compound. The embodiments and definitions described above in the context of the method of identifying an anti-aging compound, in the context of a method of preventing or treating aging, and/or in the context of a compound for use of the invention also apply to the method of determining if a patient is likely to respond to a treatment with an anti-aging compound according to the invention. Advantageously, the method of determining if a patient is likely to respond to a treatment with an anti-aging compound allows to further enhance the specificity of preventing or treating aging in a patient. Advantageously, the method of determining if a patient is likely to respond to a treatment may be used in the context of personalized medicine to determine whether a patient is likely to respond to a treatment with an anti-aging compound. In one embodiment, the anti-aging compound is a compound identified using a method of identifying an anti-aging compound according to the invention. In one embodiment, the anti-aging compound is an anti-aging compound as defined above.

Advantageously, the specificity of preventing or treating aging can be even further enhanced if a method of determining if a patient is likely to respond to a treatment further comprises
providing a sample of said patient, wherein said sample comprises senescent cells, and treating said sample with one or more anti-aging compound(s),
determining a level of phosphorylated TTP in said treated sample, and,
comparing the level of phosphorylated TTP determined in said treated sample to the level of phosphorylated TTP determined in step ii),
wherein a decreased level of phosphorylated TTP in said treated sample compared to the level of phosphorylated TTP determined in step ii) indicates that said patient is likely to respond to a treatment with said one or more anti-aging compound(s).

In one embodiment, said determining of a level of phosphorylated TTP is performed using an antibody or antigen-binding fragment thereof targeting phosphorylated TTP and/or TTP. The level of phosphorylated TTP may be efficiently determined using an antibody or antigen-binding fragment thereof.

The method of preventing or treating aging in a patient of the invention may comprise, prior to said administering a therapeutically effective amount of a compound to a patient in need thereof: performing a method of identifying an anti-aging compound and/or performing a method of determining if the patient is likely to respond to treatment with an anti-aging compound.

The term "AU-rich element" or "ARE", as used herein, refers to an adenylate-uridylate-rich element in the 3' untranslated region of a mRNA. AREs are a determinant of RNA stability, and often occur in mRNAs of proto-oncogenes, nuclear transcription factors, and cytokines. TTP is an ARE-binding protein (ARE-BP) which binds to AREs and destabilizes the mRNA. AU-rich elements typically exist in the 3'UTR, e.g. in the 3'UTR of nearly 20% of the human genes, and function in health as a fine-turning mechanism of gene expression [1]. AU-rich mRNAs may code for proteins involved in inflammation, immune response, cell cycle, apoptosis, signaling, and matrix modeling. A key RNA-binding protein that promotes AU-rich mRNA deadenylation and decay is the zinc finger protein, tristetraprolin (TTP/ZFP36). The term "TTP" or "tristetraprolin", as used herein, refers to a protein which binds to AU-rich elements (AREs) in the 3'-untranslated regions of ARE-containing mRNAs, and promotes degradation of said mRNAs. TTP is also known as zinc finger protein 36 homolog (ZFP36). In one embodiment, interactions of TTP and target mRNAs are affected by the phosphorylation state of TTP. In one embodiment, phosphorylated TTP/ZFP36 is unable to promote ARE-mRNA decay, and thus the abundance of proteins involved in inflammation and aging is increased and the half-life of these proteins is prolonged. Phosphorylation of TTP/ZFP36 by various protein kinases is one of the posttranslational modifications that may profoundly affect its cellular localization and activity [2], [3], [4]. For example, the p38/MK2 is a pathway that leads to TTP phosphorylation preventing its ability to recruit mRNA decay machinery and subsequently leading to over-production of ARE-mRNA products.

In one embodiment, phosphorylated TTP is a biomarker for detecting whether a patient is likely to respond to an anti-aging compound such as a protein kinase inhibitor and/or for detecting which anti-aging compound such as a protein kinase inhibitor will have the best therapeutic effect in a patient. The term "responding to a treatment", as used herein, relates to a therapeutic effect, particularly an anti-aging effect, being effectively evoked in a patient. In one embodiment, the therapeutic effect comprises a reduction in the level of phosphorylated TTP. In one embodiment, aging is prevented or treated in a patient by reducing a level of phosphorylated TTP in said patient. In one embodiment, the method of identifying an anti-aging compound according to the invention and/or the method of determining if a patient is likely to respond to a treatment with an anti-aging compound according to the invention is/are an in vitro method.

The terms "of the [present] invention", "in accordance with the invention", "according to the invention" and the like, as used herein are intended to refer to all aspects and embodiments of the invention described and/or claimed herein. As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### BRIEF DESCRIPTION OF THE FIGURES

The present invention is now further described by reference to the following figures.

All methods mentioned in the figure descriptions below were carried out as described in detail in the examples.

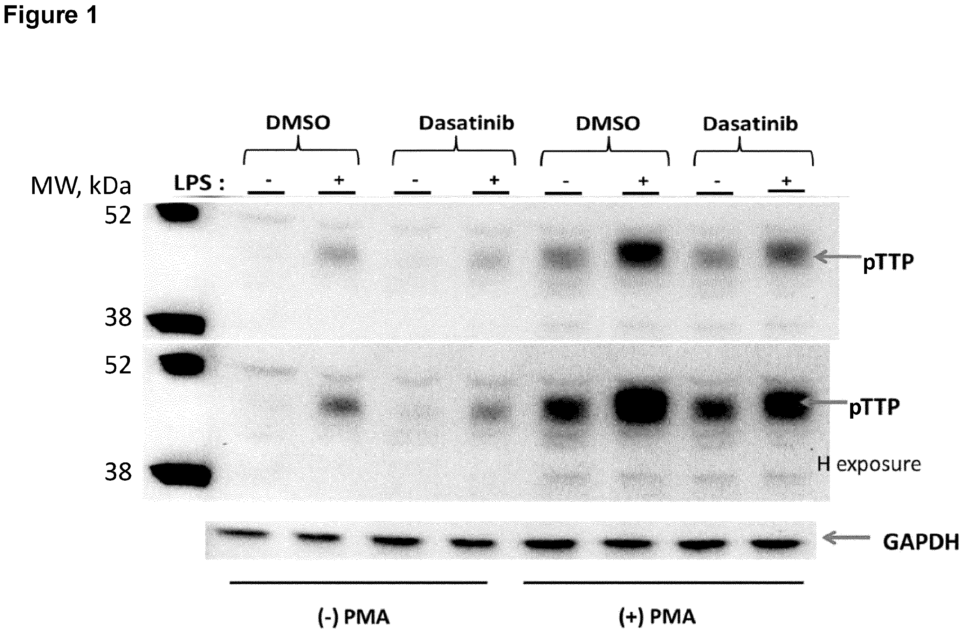

**Figure 1** shows that exemplary anti-aging compound dasatinib reduces TTP phosphorylation. LPS is an inducer of senescence in macrophages. THP-1 monocytic cells or phorbol 12-myristate 13-acetate (PMA)-differentiated THP-1 cells (macrophage type) were treated with 1 µM dasatinib for 1 hr, and then stimulated with 1 µg/ml LPS. Phosphorylated TTP leads to overproduction of mRNA and senescence-associated secretory phenotype (SASP) proteins. Advantageously, dasatinib is able to reduce phosphorylated TTP abundance.

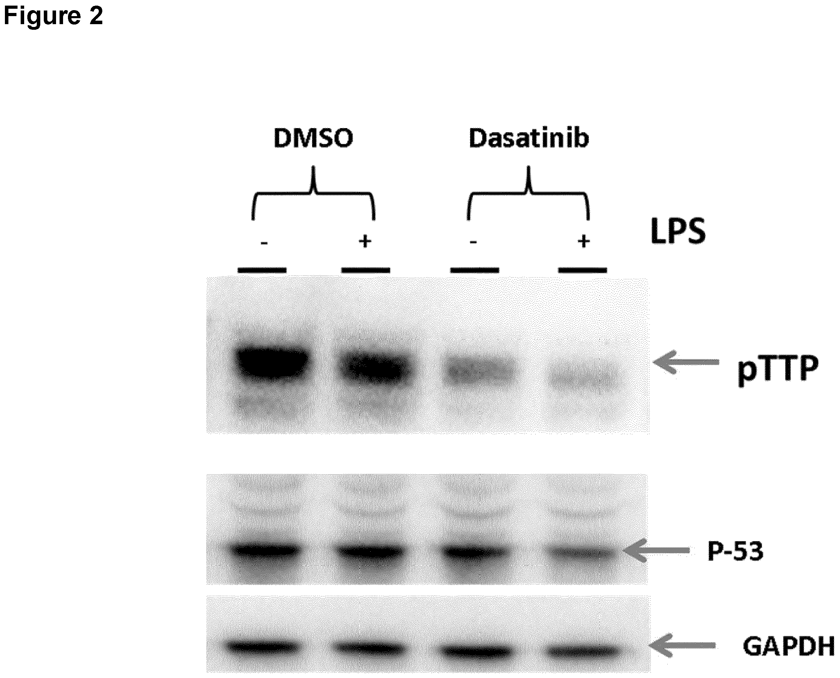

**Figure 2** shows that the exemplary senolytic compound dasatinib reduces pTTP. Moreover, p53, a senescence biomarker is also reduced by the senolytic agent dasatinib. Human macrophages generated by PMA differentiation of THP-1 cells were treated with 1 µM dasatinib and LPS was added for overnight to induce senescence.

In the following, reference is made to the examples, which are given to illustrate, not to limit the present invention.

### EXAMPLES

### Example 1:

The role of AU-rich elements-mediated mRNA stabilization in aging and how this process can be therapeutically targeted were analyzed.

*Rationale and results*: AU-rich mRNA stabilization occurs during inflammatory situations, and aging incorporates a chronic low-grade inflammatory state *inflammaging* and immunosenescence (dysfunction in immune cells caused by aging). The inventors have observed that there is a connection between aging and AU-rich mRNA stabilization. Moreover, TTP deficiency can lead to increased expression of AU-rich mRNAs that belong to inflammatory cytokines, which belong to senescence-associated secretory phenotype (SASP) genes.

### Methods:

The inventors examined (A) AU-rich mRNA stabilization during immunosenescence, and (B) the potential of senolytics in reversing this pathway.

Particularly, cellular models of inflammaging/immunosenescence were used: (a) human macrophages (differentiated THP-1 cells) and (b) bone-marrow-derived blood monocytes from WT- and TTP-KO mice. Immunosenescence in macrophages was induced by LPS endotoxin [5, 6]. Senescence was assessed by SA-β-gal staining and the markers (p53/p21/p16). For examining (A) AU-rich mRNA stabilization during immunosenescence, AU-rich mRNA decay aberrations were examined in senescent cells as opposed to normal counterparts by TTP phosphorylation, ARE-reporter assay, and mRNA half-life for SASP genes. For examining (B) the potential of senolytics in reversing this pathway, dasatinib and other senolytics (e.g., quercetin) were used to assess reversal of the perturbed ARE-mediated events using the same methods as used for (A).

Briefly, a THP-1 monocytic cell line was obtained from ATCC and cultured in complete RPMI medium with 10% FBS. It was induced by phorbol ester, PMA, 10 ng/ml to induce differentiation to normal-like macrophages. The cells were plated in 6-well plates and treated with DMSO as a control vehicle or Dasatinib 1 µM. LPS 1 µg/ml as senescence inducer for 24 hr.

For total lysates, cells were directly lysed in NP 40 lysis buffer. Lysates were sonicated to shear DNA, and equivalent lysate levels were loaded onto SDS PAGE gel, blotted to a nitrocellulose membrane, and probed with antibodies to TTP. The Affinity-purified TTP rabbit polyclonal antibody was custom-made with Genescript against the C-terminal end of TTP and was used previously to detect total TTP which is mostly phosphorylated by LPS treatment.

Differentiated THP-1 macrophages were stimulated with 1 µg/ml LPS, a senescence inducer. As shown in Figure 1, Dasatinib treatment (1 µM) led to considerable reduction in TTP phosphorylation and reduction of p53.

Outcome: It was shown that immunosenescence/SASP is linked to TTP phosphorylation leading to AU-rich mRNA stabilization. The inventors have shown that TTP phosphorylation is a biomarker of aging and can be used as a target for anti-aging drugs. Furthermore, the inventors normalized the aberrant mRNA stability and provide a drug discovery concept for screening anti-aging compounds.

The inventors have analyzed aging biology: phosphorylation & subsequent loss of TTP activity during SASP conversion in immune-senescence. The inventors have found that phosphorylated TTP may be used as biomarker or drug target, and for drug screening in aging research domain.

### Example 2:

The following table 1 shows a list of exemplary SASP markers. In one embodiment, a SASP marker is selected from the markers shown in table 1.

**Table 1: list of exemplary SASP markers which are encoded by AU-rich mRNAs.**

| **Gene Symbol** | **Definition** |
|---|---|
| ANGPT1 | angiopoietin 1 |
| AREG | amphiregulin |
| BMP2 | bone morphogenetic protein 2 |
| CCL13 | C-C motif chemokine ligand 13 |
| CCL16 | C-C motif chemokine ligand 16 |
| CCL2 | C-C motif chemokine ligand 2 |
| CCL3 | C-C motif chemokine ligand 3 |
| CCL3L1 | C-C motif chemokine ligand 3 like 1 |
| CCL4 | C-C motif chemokine ligand 4 |
| CCL7 | C-C motif chemokine ligand 7 [Source:HGNC Symbol;Acc:HGNC:10634] |
| CCL8 | C-C motif chemokine ligand 8 [Source:HGNC Symbol;Acc:HGNC:10635] |
| CD55 | CD55 molecule (Cromer blood group) [Source:HGNC Symbol;Acc:HGNC:2665] |
| CSF1 | colony stimulating factor 1 [Source:HGNC Symbol;Acc:HGNC:2432] |
| CSF2 | colony stimulating factor 2 [Source:HGNC Symbol;Acc:HGNC:2434] |
| CXCL1 | C-X-C motif chemokine ligand 1 [Source:HGNC Symbol;Acc:HGNC:4602] |
| CXCL10 | C-X-C motif chemokine ligand 10 [Source:HGNC Symbol;Acc:HGNC:10637] |
| CXCL12 | C-X-C motif chemokine ligand 12 [Source |
| CXCL2 | C-X-C motif chemokine ligand 2 [Source:HGNC Symbol;Acc:HGNC:4603] |
| CXCL3 | C-X-C motif chemokine ligand 3 [Source:HGNC Symbol;Acc:HGNC:4604] |
| CXCL8 | C-X-C motif chemokine ligand 8 [Source:HGNC Symbol;Acc:HGNC:6025] |
| EDN1 | endothelin 1 [Source:HGNC Symbol;Acc:HGNC:3176] |
| EGF | epidermal growth factor [Source:HGNC Symbol;Acc:HGNC:3229] |
| EREG | epiregulin [Source:HGNC Symbol;Acc:HGNC:3443] |
| ESM1 | endothelial cell specific molecule 1 [Source:HGNC Symbol;Acc:HGNC:3466] |
| FAS | Fas cell surface death receptor [Source:HGNC Symbol;Acc:HGNC:11920] |
| FGF2 | fibroblast growth factor 2 [Source:HGNC Symbol;Acc:HGNC:3676] |
| FGF7 | fibroblast growth factor 7 [Source:HGNC Symbol;Acc:HGNC:3685] |
| GDF15 | growth differentiation factor 15 [Source:HGNC Symbol;Acc:HGNC:30142] |
| IL6 | interleukin 6 [Source:HGNC Symbol;Acc: HGNC: 6o 18] |
| IGFBP1 | insulin like growth factor binding protein 1 [Source:HGNC Symbol;Acc:HGNC:5469] |
| IGFBP7 | insulin like growth factor binding protein 7 [Source:HGNC Symbol;Acc:HGNC:5476] |
| IL10 | interleukin 10 [Source:HGNC Symbol;Acc:HGNC:5962] |
| IL13 | interleukin 13 [Source:HGNC Symbol;Acc:HGNC:5973] |
| IL15 | interleukin 15 [Source:HGNC Symbol;Acc:HGNC:5977] |
| IL1A | interleukin 1 alpha [Source:HGNC Symbol;Acc:HGNC:5991] |
| IL1B | interleukin 1 beta [Source:HGNC Symbol;Acc:HGNC:5992] |
| IL2 | interleukin 2 [Source:HGNC Symbol;Acc:HGNC:6001] |
| IL6ST | interleukin 6 signal transducer [Source:HGNC Symbol;Acc:HGNC:6021] |
| ITGA2 | integrin subunit alpha 2 [Source:HGNC Symbol;Acc:HGNC:6137] |
| JUN | Jun proto-oncogene, AP-1 transcription factor subunit [Source:HGNC Symbol;Acc:HGNC:6204] |
| MMP13 | matrix metallopeptidase 13 [Source:HGNC Symbol;Acc:HGNC:7159] |
| MMP3 | matrix metallopeptidase 3 [Source:HGNC Symbol;Acc:HGNC:7173] |
| NAP1L4 | nucleosome assembly protein 1 like 4 [Source:HGNC Symbol;Acc:HGNC:7640] |
| PAPPA | pappalysin 1 [Source:HGNC Symbol;Acc:HGNC:86o2] |
| PLAT | plasminogen activator, tissue type [Source:HGNC Symbol;Acc:HGNC:9051] |
| PLAU | plasminogen activator, urokinase [Source:HGNC Symbol;Acc:HGNC:9052] |
| PLAUR | plasminogen activator, urokinase receptor [Source |
| PTBP1 | polypyrimidine tract binding protein 1 [Source:HGNC Symbol;Acc:HGNC:9583] |
| PTGER2 | prostaglandin E receptor 2 [Source:HGNC Symbol;Acc:HGNC:9594] |
| RPS6KA5 | ribosomal protein S6 kinase A5 [Source:HGNC Symbol;Acc:HGNC:10434] |
| SERPINE1 | serpin family E member 1 [Source:HGNC Symbol;Acc:HGNC:8583] |
| SPX | spexin hormone [Source:HGNC Symbol;Acc:HGNC:28139] |
| TIMP2 | TIMP metallopeptidase inhibitor 2 [Source:HGNC Symbol;Acc: HGNC: 11821] |
| TNF | tumor necrosis factor [Source:HGNC Symbol;Acc:HGNC:11892] |
| VEGFA | vascular endothelial growth factor A [Source:HGNC Symbol;Acc:HGNC:12680] |
| VEGFC | vascular endothelial growth factor C [Source:HGNC Symbol;Acc: HGNC: 1268 2] |
| WNT2 | Wnt family member 2 [Source:HGNC Symbol;Acc:HGNC:12780] |
| CXCL5 | C-X-C motif chemokine ligand 5 [Source:HGNC Symbol;Acc:HGNC:10642] |
| TFRC | transferrin receptor [Source:HGNC Symbol;Acc:HGNC:11763] |
| ADSS | adenylosuccinate synthase [Source:HGNC Symbol;Acc:HGNC:292] |
| PTX3 | pentraacin 3 [Source:HGNC Symbol;Acc:HGNC:9692] |
| STC1 | stanniocalcin 1 [Source:HGNC Symbol;Acc:HGNC:11373] |

### Example 3: Exemplary compounds to be tested.

The below list shows examples of compounds which can be tested in a method of identifying an anti-aging compound according to the present invention and/or a method of determining if a patient is likely to respond to a treatment with an anti-aging compound according to the present invention. Furthermore, the examples of compounds shown below are exemplary inhibitors that might be used in a method of preventing or treating aging in a patient.

For example, the compound(s) to be tested may include monoclonal antibodies such as bevacizumab, cetuximab, and ipilimumab. For example, the compound(s) to be tested may include small molecules such as bortezomib; imatinib; seliciclib; Ado-trastuzumab emtansine (Kadcyla); Afatinib (Gilotrif); Aldesleukin (Proleukin); Alectinib (Alecensa); Alemtuzumab (Campath); Atezolizumab (Tecentriq); Avelumab (Bavencio); Axitinib (Inlyta); Belimumab (Benlysta); Belinostat (Beleodaq); Bevacizumab (Avastin); Blinatumomab (Blincyto); Bortezomib (Velcade); Bosutinib (Bosulif); Brentuximab vedotin (Adcetris); Brigatinib (Alunbrig); Cabozantinib (Cabometyx [tablet], Cometriq [capsule]); Canakinumab (Ilaris); Carfilzomib (Kyprolis); Ceritinib (Zykadia); Cetuximab (Erbitux); Cobimetinib (Cotellic); Crizotinib (Xalkori); Dabrafenib (Tafinlar); Daratumumab (Darzalex); Dasatinib (Sprycel); Denosumab (Xgeva); Dinutuximab (Unituxin); Durvalumab (Imfinzi); Elotuzumab (Empliciti); Enasidenib (Idhifa); Erlotinib (Tarceva); Everolimus (Afinitor); Gefitinib (Iressa); Ibritumomab tiuxetan (Zevalin); Ibrutinib (Imbruvica); Idelalisib (Zydelig); Imatinib (Gleevec); Ipilimumab (Yervoy); Ixazomib (Ninlaro); Lapatinib (Tykerb); Lenvatinib (Lenvima); Midostaurin (Rydapt); Necitumumab (Portrazza); Neratinib (Nerlynx); Nilotinib (Tasigna); Niraparib (Zejula); Nivolumab (Opdivo); Obinutuzumab (Gazyva); Ofatumumab (Arzerra, HuMax-CD20); Olaparib (Lynparza); Olaratumab (Lartruvo); Osimertinib (Tagrisso); Palbociclib (Ibrance); Panitumumab (Vectibix); Panobinostat (Farydak); Pazopanib (Votrient); Pembrolizumab (Keytruda); Pertuzumab (Perjeta); Ponatinib (Iclusig); Ramucirumab (Cyramza); Regorafenib (Stivarga); Ribociclib (Kisqali); Rituximab (Rituxan, Mabthera); Rituximab/hyaluronidase human (Rituxan Hycela); Romidepsin (Istodax); Rucaparib (Rubraca); Ruxolitinib (Jakafi); Siltuximab (Sylvant); Sipuleucel-T (Provenge); Sonidegib (Odomzo); Sorafenib (Nexavar); Temsirolimus (Torisel); Tocilizumab (Actemra); Tofacitinib (Xeljanz); Tositumomab (Bexxar); Trametinib (Mekinist); Trastuzumab (Herceptin); Vandetanib (Caprelsa); Vemurafenib (Zelboraf); Venetoclax (Venclexta); Vismodegib (Erivedge); Vorinostat (Zolinza); and Ziv-aflibercept (Zaltrap).

Alternatively or additionally, the compounds to be tested may include the compounds listed in example 4.

### Example 4: Exemplary protein kinase inhibitors.

The list below shows examples of protein kinase inhibitors which can be tested in a method of identifying an anti-aging compound according to the present invention and/or a method of determining if a patient is likely to respond to a treatment with an anti-aging compound according to the present invention. Furthermore, the examples of protein kinase inhibitors shown below are exemplary inhibitors that might be used in a method of preventing or treating aging in a patient.

For example, the protein kinase inhibitor may be selected from (-)-BAY-1251152; (-)-Indolactam V; (+)-BAY-1251152; (±)-Zanubrutinib; (1S,3R,5R)-PIM447 (dihydrochloride); (3S,4S)-Tofacitinib; (E)-AG 99; (E)-Necrosulfonamide; [6]-Gingerol; 1,2,3,4,5,6-Hexabromocyclohexane; 1,3-Dicaffeoylquinic acid; 1-Azakenpaullone; 1-Naphthyl PP1; 1-NM-PP1; 2,5-Dihydroxybenzoic acid; 2-D08; 2-Deoxy-D-glucose; 2-Methoxy-1,4-naphthoquinone; 2-Phospho-L-ascorbic acid trisodium salt; 3,4-Dimethoxycinnamic acid; 3BDO; 3-Bromopyruvic acid; 3-Methyladenine (3-MA); 4µ8C; 5-Aminosalicylic Acid; 5-Bromoindole; 5-Iodotubercidin; 6-(Dimethylamino)purine; 6-Bromo-2-hydroxy-3-methoxybenzaldehyde; 7,8-Dihydroxyflavone; 7-Hydroxy-4-chromone; 7-Methoxyisoflavone; 8-Bromo-cAMP sodium salt; A 419259 (trihydrochloride); A 77-01; A 83-01 sodium salt; A-443654; A-484954; A66; A-674563; A-769662; ABBV-744; Abemaciclib; Abrocitinib; ABT-702 dihydrochloride; AC480 (BMS-599626); AC710; Acalabrutinib (ACP-196); Acalisib; acalisib (GS-9820); ACHP (Hydrochloride); ACTB-1003; Acumapimod; AD80; Adavosertib; AEE788; Afatinib; Afatinib (BIBW2992); Afatinib (dimaleate); Afuresertib; AG 555; AG-1024; AG126; AG-1478; AG-18; AG-490; Agerafenib; AGL-2263; AICAR; AIM-100; AKT inhibitor VIII; AKT Kinase Inhibitor; Akt1 and Akt2-IN-1; Akti-1/2; Alectinib; Alisertib (MLN8237); ALK inhibitor 1; ALK inhibitor 2; ALK-IN-1; Allitinib tosylate; Alofanib; Alpelisib; Altiratinib; ALW-II-41-27; AM-2394; Amcasertib (BBI503); AMG 337; AMG 900; AMG 925 (HCl); AMG-208; AMG319; AMG-337; AMG-3969; AMG-458; AMG-47a; AMG-900; Amlexanox; Amuvatinib (MP-470); ANA-12; Anacardic Acid; Anlotinib (AL3818) dihydrochloride; AP26113-analog (ALK-IN-1); Apatinib; Apatinib mesylate; Apigenin; Apitolisib; APS-2-79; APY0201; APY29; AR-A014418; ARN-3236; ARQ 531; AS-252424; AS601245; AS-604850; AS-605240; Asciminib; Asciminib (ABL001); ASP3026; ASP5878; AST 487; AST-1306; Astragaloside IV; AT13148; AT7519; AT7867; AT9283; Atuveciclib; Atuveciclib S-Enantiomer; Aurora A inhibitor I; Autophinib; AUZ 454; AV-412; Avapritinib; Avitinib (maleate); AX-15836; Axitinib; AZ 3146; AZ 628; AZ 960; AZ1495; AZ191; AZ20; AZ-23; AZ304; AZ31; AZ3146; AZ32; AZ5104; AZ960; Azaindole 1; AZD 6482; AZD0156; AZD-0364; AZD1080; AZD1152; AZD1208; AZD1390; AZD-1480; AZD2858; AZD2932; AZD3229; AZD3264; AZD3463; AZD-3463; AZD3759; AZD4547; AZD4573; AZD5363; AZD5438; AZD-5438; AZD6482; AZD6738; AZD7507; AZD7545; AZD7762; AZD-7762; AZD8055; AZD-8055; AZD8186; AZD8330; AZD8835; AZD-8835; AZM475271; Bafetinib (INNO-406); Bakuchiol; Barasertib-HQPA; Bardoxolone Methyl; Baricitinib; BAW2881 (NVP-BAW2881); BAY 11-7082; Bay 11-7085; BAY 1217389; BAY 1895344 (BAY-1895344); Bay 65-1942 (hydrochloride); BAY1125976; BAY1217389; BAY-1895344 (hydrochloride); BAY-61-3606; BDP5290; BEBT-908; Belizatinib; Bemcentinib; Bentamapimod; Berbamine (dihydrochloride); Berberine (chloride hydrate); Berzosertib; BF738735; BFH772; BGG463; BGT226 (NVP-BGT226); BI 2536; BI-4464; BI605906; BI-78D3; BI-847325; BIBF 1202; BIBF0775; BI-D1870; Bikinin; Bimiralisib; Binimetinib; Binimetinib (MEK162, ARRY-162, ARRY-438162); BIO; BIO-acetoxime; Biochanin A; Bisindolylmaleimide I; Bisindolylmaleimide I (GF109203X); Bisindolylmaleimide IX (Ro 31-8220 Mesylate); BIX 02188; BIX 02189; BIX02188; BIX02189; BLU-554 (BLU554); BLU9931; BLZ945; BMS 777607; BMS-265246; BMS-345541; BMS-5; BMS-509744; BMS-536924; BMS-582949; BMS-690514; BMS-754807; BMS-777607; BMS-794833; BMS-911543; BMS-935177; BMS-986142; BMS-986195; BMX-IN-1; BOS-172722; Bosutinib (SKI-606); BPR1J-097 Hydrochloride; bpV (HOpic); BQR-695; B-Raf IN 1; BRAF inhibitor; B-Raf inhibitor 1; Brivanib; Brivanib (BMS-540215); Brivanib Alaninate (BMS-582664); BS-181; BTK IN-1; Btk inhibitor 1; BTK inhibitor 1 (Compound 27); Btk inhibitor 1 (R enantiomer); Btk inhibitor 2; Bucladesine (calcium salt); Bucladesine (sodium salt); Buparlisib; Butein; BX517; BX795; BX-795; BX-912; Ca2+ channel agonist 1; CA-4948; Cabozantinib; Cabozantinib (S-malate); Cabozantinib (XL184, BMS-907351); Cabozantinib malate (XL184); CAL-130 (Hydrochloride); CaMKII-IN-1; Canertinib (CI-1033); Capivasertib; Capmatinib; Casein Kinase II Inhibitor IV; CAY10505; CC-115; CC-223; CC-401 (hydrochloride); CC-671; CC-90003; CCG215022; CCT 137690; CCT020312; CCT128930; CCT129202; CCT137690; CCT196969; CCT241533 (hydrochloride); CCT241736; CCT245737; CCT-251921; CDK9-IN-1; CDK9-IN-2; CDKI-73; CDK-IN-2; Cediranib; Cediranib Maleate; Centrinone; Centrinone-B; CEP-28122 (mesylate salt); CEP-32496; CEP-33779; CEP-37440; CEP-40783; Ceralasertib; Cerdulatinib; Cerdulatinib (PRT062070, PRT2070); Ceritinib; Ceritinib dihydrochloride; CFI-400945; CFI-402257 hydrochloride; cFMS Receptor Inhibitor II; c-Fms-IN-2; CG-806; CGI1746; CGI-1746; CGK 733; CGK733; CGP 57380; CGP60474; CH5132799; CH5183284; CH5183284 (Debio-1347); CH7057288; Chelerythrine Chloride; CHIR-124; CHIR-98014; CHIR-99021; CHIR-99021 (CT99021); Chk2 Inhibitor II (BML-277); Chloropyramine hydrochloride; CHMFL-BMX-078; CHR-6494; Chroman 1; Chrysophanic Acid; CHZ868; CI-1040; CID 2011756; CID755673; CK1-IN-1; c-Kit-IN-1; CL-387785; CL-387785 (EKI-785); CLK1-IN-1; c-Met inhibitor 1; CNX-2006; CNX-774; Cobimetinib; Cobimetinib (GDC-0973, RG7420); Cobimetinib (hemifumarate); Cobimetinib (racemate); Compound 401; Corynoxeine; CP21R7; CP21R7 (CP21); CP-466722; CP-673451; CP-724714; Crenolanib; Crizotinib; CRT0066101; CRT0066101 dihydrochloride; CT7001 hydrochloride; Cucurbitacin E; Cucurbitacin I; CUDC-101; CUDC-907; CVT-313; CX-6258; Cyasterone; CYC065; CYC116; CZ415; CZC24832; CZC-25146; CZC-54252; CZC-8004; D 4476; D4476; Dabrafenib; Dabrafenib (GSK2118436); Dabrafenib (Mesylate); Dabrafenib Mesylate; Dacomitinib; Dacomitinib (PF299804, PF299); Dactolisib (Tosylate); Danthron; Danusertib; Danusertib (PHA-739358); Daphnetin; Dasatinib; Dasatinib Monohydrate; DB07268; DCC-2618; DCP-LA; DDR1-IN-1; Decernotinib (VX-509); Defactinib; Degrasyn; Deguelin; Dehydrocorydaline (chloride); Dehydrocostus Lactone; DEL-22379; Delcasertib; Delgocitinib; Derazantinib; Derazantinib(ARQ-087); Dicoumarol; Dihexa; Dihydromyricetin; Dilmapimod; Dinaciclib; Dinaciclib (SCH727965); DMAT; DMH1; DMH-1; Doramapimod; Doramapimod (BIRB 796); Dorsomorphin (Compound C); Dorsomorphin (dihydrochloride); Dovitinib; Dovitinib (lactate); Dovitinib (TKI-258) Dilactic Acid; Dovitinib (TKI258) Lactate; Dovitinib (TKI-258, CHIR-258); DPH; Dubermatinib; Duvelisib; Duvelisib (R enantiomer); EAI045; eCF506; Edicotinib; eFT-508 (eFT508); EG00229; EGFR-IN-3; Ellagic acid; EMD638683; EMD638683 (R-Form); EMD638683 (S-Form); Emodin; Empesertib; Encorafenib; ENMD-2076; ENMD-2076 L-(+)-Tartaric acid; Entospletinib; Entospletinib (GS-9973); Entrectinib; Entrectinib (RXDX-101); Enzastaurin; Enzastaurin (LY317615); Erdafitinib; Erdafitinib (JNJ-42756493); ERK5-IN-1; Erlotinib; ETC-1002; ETC-206; ETP-46321; ETP-46464; Everolimus (RAD001); Evobrutinib; EX229; Falnidamol; Fasudil (Hydrochloride); Fedratinib; Fenebrutinib; Ferulic acid; Ferulic acid methyl ester; FGF401; FGFR4-IN-1; FIIN-2; FIIN-3; Filgotinib; Filgotinib (GLPG0634); Fimepinostat; Fingolimod; Fisogatinib; Flavopiridol; FLLL32; FLT3-IN-1; FLT3-IN-2; Flufenamic acid; Flumatinib; Flumatinib (mesylate); FM381; FM-381; FMK; FN-1501; Foretinib; Foretinib (GSK1363089); Formononetin; Fostamatinib (R788); FR 180204; FRAX1036; FRAX486; FRAX597; Fruquintinib; Futibatinib; G-5555; G-749; Galunisertib; Gambogenic acid; Gandotinib; Gandotinib (LY2784544); GDC-0077; GDC-0084; GDC-0326; GDC-0339; GDC-0349; GDC-0575 (ARRY-575, RG7741); GDC-0623; GDC-0834; GDC-0834 (Racemate); GDC-0834 (S-enantiomer); GDC-0879; Gedatolisib (PF-05212384, PKI-587); Gefitinib; Gefitinib (ZD1839); Genistein; Gilteritinib (ASP2215); Ginkgolide C; Ginsenoside Rb1; Ginsenoside Re; Glesatinib (hydrochloride); GLPG0634 analog; GNE-0877; GNE-317; GNE-477; GNE-493; GNE-7915; GNE-9605; GNF-2; GNF-5; GNF-5837; GNF-7; Go 6983; G06976; Golvatinib (E7050); GSK 3 Inhibitor IX; GSK 650394; GSK1059615; GSK1070916; GSK180736A; GSK180736A (GSK180736); GSK1838705A; GSK1904529A; GSK2110183 (hydrochloride); GSK2256098; GSK2292767; GSK2334470; GSK2578215A; GSK2606414; GSK2636771; GSK2656157; GSK269962A; GSK2850163; GSK2982772; GSK-3 inhibitor 1; GSK429286A; GSK461364; GSK481; GSK'481; GSK'547; GSK583; GSK650394; GSK690693; GSK-872; GSK'963; Gusacitinib; GW 441756; GW 5074; GW2580; GW441756; GW5074; GW788388; GW843682X; GZD824; GZD824 Dimesylate; H3B-6527; H-89 (dihydrochloride); HA-100; Harmine; Harmine hydrochloride; HER2-Inhibitor-1; Hesperadin; HG-10-102-01; HG-14-10-04; HG6-64-1; HG-9-91-01; Hispidulin; HMN-214; Honokiol; HS-10296 hydrochloride; HS-1371; HS-173; HTH-01-015; hVEGF-IN-1; Hydroxyfasudil; Ibrutinib; Ibrutinib (PCI-32765); IC261; IC-87114; Icotinib; ID-8; Idelalisib; Idelalisib (CAL-101, GS-1101); IITZ-01; IKK 16; IKK-IN-1; Ilginatinib; IM-12; Imatinib; Imatinib Mesylate (STI571); IMD 0354; IMD-0354; IMD-0560; INCB053914 (phosphate); Indirubin; Indirubin-3'-monoxime; Infigratinib; Ingenol; INH14; IPA-3; Ipatasertib; IPI-3063; IPI549; IPI-549; IQ-1S (free acid); IRAK inhibitor 1; IRAK inhibitor 2; IRAK inhibitor 4 (trans); IRAK inhibitor 6; IRAK-1-4 Inhibitor I; IRAK4-IN-1; Irbinitinib (ARRY-380, ONT-380); ISCK03; Isorhamnetin; Isorhamnetin 3-O-neohesperoside; Isovitexin; ISRIB (transisomer); Itacitinib; ITD-1; ITX5061; JAK3-IN-1; JANEX-1; JH-II-127; JH-VIII-157-02; JI-101; JNJ-38877605; JNJ-38877618; JNJ-47117096 hydrochloride; JNJ-7706621; JNK Inhibitor IX; JNK-IN-7; JNK-IN-8; K02288; K03861; K145 (hydrochloride); kb NB 142-70; KD025 (SLx-2119); KDU691; Kenpaullone; Ki20227; Ki8751; kira6; KN-62; KN-92 (hydrochloride); KN-93; KN-93 Phosphate; KPT-9274; KRN 633; KU-0063794; KU-55933; KU-57788; KU-60019; KW-2449; KX1-004; KX2-391; L-779450; Lapatinib; Larotrectinib (LOXO-101) sulfate; Larotrectinib sulfate; Lazertinib; Lazertinib (YH25448,GNS-1480); Lck Inhibitor; Lck inhibitor 2; LDC000067; LDC1267; LDC4297; LDN-193189 2HCl; LDN-212854; LDN-214117; Leflunomide; Leniolisib; Lenvatinib; Lerociclib dihydrochloride; LFM-A13; Lifirafenib; Linifanib; Linsitinib; LJH685; LJI308; L-Leucine; LM22A-4; LM22B-10; Longdaysin; Lonidamine; Lorlatinib; Lorlatinib?(PF-6463922); Losmapimod; Losmapimod (GW856553X); Loureirin B; LRRK2 inhibitor 1; LRRK2-IN-1; LSKL, Inhibitor of Thrombospondin (TSP-1); LTURM34; Lucitanib; Lupeol; LX2343; LXH254; LXS196; LY2090314; LY2109761; LY2409881; LY2584702; LY2584702 Tosylate; LY2608204; LY2857785; LY2874455; LY294002; LY3009120; LY3023414; LY3177833; LY3200882; LY3214996; LY3295668; LY364947; LY-364947; LYN-1604 hydrochloride; Magnolin; Masitinib; MBQ-167; MC180295; MCB-613; MEK inhibitor; MELK-8a (hydrochloride); Merestinib; Mesalamine; Metadoxine; Metformin (hydrochloride); Methylthiouracil; MGCD-265 analog; MHP; MHY1485; Midostaurin; Milciclib (PHA-848125); Miltefosine; Miransertib; Mirin; Mirk-IN-1; Mitoxantrone; MK 2206 (dihydrochloride); MK-2461; MK2-IN-1 (hydrochloride); MK-3903; MK-5108; MK-8033; MK8722; MK-8745; MK-8776 (SCH 900776); MKC3946; MKC8866; MKC9989; ML167; ML347; ML-7 HCl; MLi-2; MLN0905; MLN120B; MLN2480; MLN8054; MNS; MNS (3,4-Methylenedioxy-β-nitrostyrene, MDBN); Momelotinib; Motesanib; MP7; MP-A08; MPI-0479605; Mps1-IN-1; Mps1-IN-2; MRT67307 HCl; MRT68921 (hydrochloride); MRX-2843; MSC2530818; MSDC 0160; mTOR inhibitor-3; MTX-211; Mubritinib; Mutated EGFR-IN-1; Myricetin; NAMI-A; Naquotinib(ASP8273); Narciclasine; Nazartinib; Nazartinib (EGF816, NVS-816); NCB-0846; Nec-1s (7-Cl-O-Nec1); Necrostatin-1; Necrosulfonamide; Nedisertib; Neflamapimod; Nemiralisib; Neohesperidin dihydrochalcone; Neratinib (HKI-272); NG 52; NH125; Nilotinib; Nilotinib (AMN-107); Ningetinib; Nintedanib; NMS-P937 (NMS1286937); Nocodazole; Norcantharidin; Notoginsenoside R1; NPS-1034; NQDI-1; NSC 228155; NSC 42834; NSC12; NSC781406; NT157; NU 7026; NU2058; NU6027; NU6300; NU7026; NU7441 (KU-57788); NVP-2; NVP-ACC789; NVP-ADW742; NVP-BAW2881; NVP-BHG712; NVP-BHG712 isomer; NVP-BSK805 2HCl; NVP-BVU972; NVP-LCQ195; NVP-TAE 226; NVP-TAE 684; NVS-PAK1-1; Oclacitinib (maleate); Oglufanide; Olmutinib; Omipalisib; Omtriptolide; ON123300; ONO-4059 (GS-4059) hydrochloride; Orantinib (TSU-68, SU6668); Oridonin; OSI-027; OSI-420; OSI-930; Osimertinib; OSU-03012 (AR-12); OTS514 hydrochloride; OTS964; OTSSP167 (hydrochloride); P276-00; p38α inhibitor 1; p38-α MAPK-IN-1; Pacritinib; Palbociclib (hydrochloride); Palbociclib (isethionate); Palomid 529; Palomid 529 (P529); Pamapimod; Parsaclisib; Pazopanib; PCI 29732; PCI-33380; PD 169316; PD0166285; PD0325901; PD153035; PD158780; PD-166866; PD168393; PD173074; PD173955; PD184352 (CI-1040); PD318088; PD98059; Peficitinib; Pelitinib; Pelitinib (EKB-569); Pemigatinib; Perifosine (KRX-0401); Pexidartinib; Pexmetinib (ARRY-614); PF-00562271 Besylate; PF-03814735; PF-04217903; PF-04217903 (methanesulfonate); PF-04691502; PF-04965842; PF-05231023; PF-06273340; PF-06409577; PF-06447475; PF-06459988; PF06650833; PF-06651600; PF-06700841 (P-Tosylate); PF-3758309; PF-431396; PF-4708671; PF-477736; PF-4800567; PF-4989216; PF-543 (Citrate); PF-562271; PF-573228; PFK15; PFK158; PH-797804; PHA-665752; PHA-680632; PHA-767491; PHA-793887; Phenformin (hydrochloride); Phorbol 12-myristate 13-acetate; PHT-427; PI-103; PI-103 (Hydrochloride); PI-3065; PI3K-IN-1; PI3Kδ-IN-2; PI4KIII beta inhibitor 3; Piceatannol; Picfeltarraenin IA; Picropodophyllin; Pictilisib (GDC-0941); PIK-293; PIK-294; PIK-75; PIK-75 HCl; PIK-93; PIK-III; Pilaralisib; Pilaralisib analogue; Pim1/AKK1-IN-1; PIM-447 (dihydrochloride); Pimasertib; Pitavastatin Calcium; PKC-IN-1; PKC-theta inhibitor; PKM2 inhibitor(compound 3k); Pluripotin; PLX-4720; PLX647; PLX7904; PLX8394; PND-1186; PND-1186 (VS-4718); Poloxime; Poloxin; Ponatinib (AP24534); Poziotinib (HM781-36B); PP1; PP121; PP2; PQ 401; PQR620; Prexasertib; PRN1008; PRN1371; PRN694; PROTAC CDK9 Degrader-1; Protein kinase inhibitors 1 hydrochloride; PRT-060318; PRT062607 (Hydrochloride); PS-1145; Psoralidin; Purvalanol A; Purvalanol B; PYR-41; Pyridone 6; Pyrotinib dimaleate; Quercetin; Quizartinib (AC220); R112; R1487 (Hydrochloride); R1530; R-268712; R406; R406 (free base); R547; R788 (Fostamatinib) Disodium; Rabusertib (LY2603618); Radotinib; RAF265; RAF265 (CHIR-265); RAF709; Ralimetinib (LY2228820); Rapamycin (Sirolimus); Ravoxertinib; Rebastinib; Refametinib; Refametinib (RDEA119, Bay 86-9766); Regorafenib; Repotrectinib; RepSox; Resveratrol; Reversine; RG13022; RG14620; RGB-286638 (free base); Ribociclib; Ridaforolimus (Deforolimus, MK-8669); Rigosertib (ON-01910); Rigosertib (sodium); Rimacalib; RIP2 kinase inhibitor 1; RIP2 kinase inhibitor 2; RIPA-56; Ripasudil; Ripretinib; RK-24466; RKI-1447; RN486; Ro 28-1675; Ro 5126766; Ro3280; Ro-3306; RO4987655; RO9021; Roblitinib; Rociletinib; Rociletinib (CO-1686, AVL-301); Rociletinib hydrobromide; Rogaratinib; Roscovitine (Seliciclib,CYC202); Rosmarinic acid; Ruboxistaurin (LY333531 HCl); Ruxolitinib; Ruxolitinib (phosphate); Ruxolitinib (S enantiomer); RXDX-106 (CEP-40783); S49076; SAFit2; Salidroside; Salubrinal; Sapanisertib; Sapitinib; SAR-020106; SAR125844; SAR131675; SAR-20347; SAR-260301; SAR405; SAR407899; Saracatinib; Saracatinib (AZD0530); Savolitinib; Savolitinib(AZD6094, HMPL-504); SB 202190; SB 203580; SB 203580 (hydrochloride); SB 239063; SB 242235; SB 415286; SB 525334; SB1317; SB202190 (FHPI); SB203580; SB216763; SB239063; SB415286; SB431542; SB-431542; SB505124; SB-505124; SB525334; SB590885; SB-590885; SBE 13 HCl; SBI-0206965; SC-514; SC66; SC79; SCH-1473759 (hydrochloride); SCH772984; SCH900776; Schisandrin B (Sch B); Scopoletin; SCR-1481B1; Scutellarein; Scutellarin; SD 0006; SD-208; SEL120-34A (monohydrochloride); Seletalisib; Seletalisib (UCB-5857); Seliciclib; Selitrectinib; Selonsertib (GS-4997); Selumetinib; Selumetinib (AZD6244); Semaxanib (SU5416); Semaxinib; Senexin A; Sennoside B; Serabelisib; Serabelisib (INK-1117,MLN-1117,TAK-117); SF1670; SF2523; SGI-1776; SGI-1776 free base; SGI-7079; SGX-523; Silmitasertib; Simurosertib; Sitravatinib; Sitravatinib (MGCD516); SJ000291942; SK1-IN-1; Skatole; Skepinone-L; SKF-86002; SKI II; SKLB1002; SKLB4771; SL327; SL-327; SLV-2436; SLx-2119; SM 16; SMI-16a; SMI-4a; SNS-032; SNS-032 (BMS-387032); SNS-314; SNS-314 Mesylate; Sodium dichloroacetate (DCA); Sodium Monofluorophosphate; Solanesol (Nonaisoprenol); Solcitinib; Sorafenib; Sorafenib Tosylate; Sotrastaurin; SP600125; Spebrutinib; SPHINX31; SR-3029; SR-3306; SR-3677; Src Inhibitor 1; SRPIN340; S-Ruxolitinib (INCB018424); SSR128129E; Staurosporine; STF-083010; STO-609; SU 5402; SU11274; SU14813; SU14813 (maleate); SU1498; SU5402; SU5408; SU6656; SU9516; Sulfatinib; SUN11602; Sunitinib; Sunitinib Malate; T56-LIMKi; TA-01; TA-02; TAE226 (NVP-TAE226); TAE684 (NVP-TAE684); TAK-285; TAK-580; TAK-593; TAK-632; TAK-659; TAK-715; TAK-733; TAK-901; TAK-960; Takinib; Talmapimod; Tandutinib; Tandutinib (MLN518); Tanzisertib; Tanzisertib(CC-930); tarloxotinib bromide; TAS-115 mesylate; TAS-301; TAS6417; Taselisib; Tat-NR2B9c; Tat-NR2B9c (TFA); Tauroursodeoxycholate (Sodium); Tauroursodeoxycholate dihydrate; Taxifolin (Dihydroquercetin); TBB; TBK1/IKKε-IN-2; TC13172; TC-DAPK 6; TCS 359; TCS JNK 5a; TCS PIM-1 1; TCS-PIM-1-4a; TDZD-8; Telatinib; Temsirolimus (CCI-779, NSC 683864); Tenalisib; Tenalisib (RP6530); Tepotinib; Tepotinib (EMD 1214063); TG 100572 (Hydrochloride); TG003; TG100-115; TG100713; TG101209; TGX-221; Theliatinib (HMPL-309); Thiazovivin; THZ1; THZ1-R; THZ2; THZ531; TIC10; TIC10 Analogue; Tideglusib; Tie2 kinase inhibitor; Tirabrutinib; Tirbanibulin (Mesylate); Tivantinib; Tivantinib (ARQ 197); Tivozanib; Tivozanib (AV-951); Toceranib phosphate; Tofacitinib; Tofacitinib (CP-690550,Tasocitinib); Tolimidone; Tomivosertib; Torin 1; Torin 2; Torkinib; Tozasertib (VX-680, MK-0457); TP0427736 HCl; TP-0903; TP-3654; TPCA-1; TPPB; TPX-0005; Trametinib; trans-Zeatin; Trapidil; Triciribine; TTP 22; Tucatinib; TWS119; TyK2-IN-2; Tyk2-IN-4; Tyrosine kinase inhibitor; Tyrosine kinase-IN-1; Tyrphostin 23; Tyrphostin 9; Tyrphostin A9; Tyrphostin AG 1296; Tyrphostin AG 528; Tyrphostin AG 879; U0126; U0126-EtOH; UCB9608; UK-371804 HCl; Ulixertinib; ULK-101; UM-164; Umbralisib; Umbralisib R-enantiomer; UNC2025; UNC2881; Upadacitinib; Uprosertib; URMC-099; Vactosertib; Vactosertib (Hydrochloride); Valrubicin; Vandetanib; Varlitinib; Vatalanib (PTK787) 2HCl; VE-821; VE-822; Vecabrutinib; Vemurafenib; VER-246608; Verbascoside; Vistusertib; Volasertib (BI 6727); VO-Ohpic trihydrate; Voxtalisib; VPS34 inhibitor 1 (Compound 19, PIK-III analogue); Vps34-IN-1; Vps34-IN-2; Vps34-PIK-III; VS-5584; VS-5584 (SB2343); VTX-27; VX-ne; VX-702; VX-745; WAY-600; Wedelolactone; WEHI-345; WH-4-023; WHI-P154; WHI-P180; WHI-P97; WNK463; Wogonin; Wortmannin; WP1066; WYE-125132 (WYE-132); WYE-132; WYE-354; WZ3146; WZ-3146; WZ4002; WZ4003; WZ8040; X-376; XL019; XL147 analogue; XL228; XL388; XL413 (BMS-863233); XMD16-5; XMD17-109; XMD8-87; XMD8-92; Y15; Y-27632; Y-33075; Y-39983 HCl; YKL-05-099; YLF-466D; YM-201636; YU238259; Zanubrutinib; ZD-4190; ZINC00881524; ZINC00881524 (ROCK inhibitor); ZLN024 (hydrochloride); ZM 306416; ZM 323881 HCl; ZM 336372; ZM 39923 HCl; ZM 447439; ZM39923 (hydrochloride); ZM-447439; Zotarolimus(ABT-578); and ZSTK474.

### REFERENCES

[1] T. Bakheet, E. Hitti, K. Khabar, ARED-Plus: an updated and expanded database of AU-rich element-containing mRNAs and pre-mRNAs. Nucleic acids research 46, D218-D220 (2018).
[2] Brook, M., C. R. Tchen, T. Santalucia, J. McIlrath, J. S. Arthur, J. Saklatvala and A. R. Clark (2006). "Posttranslational regulation of tristetraprolin subcellular localization and protein stability by p38 mitogen-activated protein kinase and extracellular signal-regulated kinase pathways." Mol Cell Biol 26(6): 2408-2418.
[3] Hitti, E., T. Iakovleva, M. Brook, S. Deppenmeier, A. D. Gruber, D. Radzioch, A. R. Clark, P. J. Blackshear, A. Kotlyarov and M. Gaestel (2006). "Mitogen-activated protein kinase-activated protein kinase 2 regulates tumor necrosis factor mRNA stability and translation mainly by altering tristetraprolin expression, stability, and binding to adenine/uridine-rich element." Mol Cell Biol 26(6): 2399-2407.
[4] Vlasova-St Louis, I. and P. R. Bohjanen (2016). "Feedback Regulation of Kinase Signaling Pathways by AREs and GREs." Cells 5(1): 4.
[5] L. Mahmoud, A. S. Abdulkarim, S. Kutbi, W. Moghrabi, S. Altwijri, K. Khabar, E. G. Hitti, Post-Transcriptional Inflammatory Response to Intracellular Bacterial c-di-AMP. Frontiers in immunology 10, 3050 (2019).
[6] H. Wang, H. Fu, R. Zhu, X. Wu, X. Ji, X. Li, H. Jiang, Z. Lin, X. Tang, S. Sun, J. Chen, X. Wang, Q. Li, Y. Ji, H. Chen, BRD4 contributes to LPS-induced macrophage senescence and promotes progression of atherosclerosis-associated lipid uptake. Aging 12, 9240-9259 (2020).

The features of the present invention disclosed in the specification, the claims, and/or in the accompanying figures may, both separately and in any combination thereof, be material for realizing the invention in various forms thereof.

## Claims

1. A method of identifying an anti-aging compound comprising, in any order, the following steps:
a) providing a sample comprising senescent cells, optionally a tissue sample,
b) optionally, determining a level of phosphorylated TTP in said sample,
c) providing one or more compound(s) to be tested,
d) treating said sample with said one or more compound(s),
e) determining whether said one or more compound(s) reduce(s) the levels of phosphorylated TTP in said treated sample compared to a control,
wherein a reduction in the level of phosphorylated TTP indicates that said one or more compound(s) is/are an anti-aging compound.

2. The method according to claim 1, wherein said control in step e) is a level of phosphorylated TTP determined in step b), and/or is a reference value, and/or is a level of phosphorylated TTP determined in a reference sample.

3. The method according to claim 1 or 2, wherein said reduction is a reduction by at least 15 %, preferably by at least 20 %, more preferably by at least 25 %.

4. The method according to any one of the foregoing claims, wherein said level of phosphorylated TTP is determined using an antibody or antigen-binding fragment thereof targeting phosphorylated TTP.

5. The method according to any one of the foregoing claims, wherein the one or more compound(s) is/are selected from small molecule inhibitors, antibodies, and antigen-binding fragments thereof; wherein, preferably, the one or more compound(s) is/are selected from protein kinase inhibitors.

6. The method according to any one of the foregoing claims, wherein the anti-aging compound is a senolytic compound, a senomorphic compound, and/or a senotherapeutic compound.

7. The method according to any one of the foregoing claims, wherein the method further comprises a step of determining whether said one or more compound(s) reduce(s) a senescence-associated secretory phenotype, a level of at least one senescence-associated secretory phenotype protein, and/or a gene expression of at least one senescence-associated secretory phenotype gene, in said treated sample compared to said control;
wherein, optionally,
said determining whether said one or more compound(s) reduce(s) a senescence-associated secretory phenotype (SASP) comprises a SA-β-gal staining and/or measuring a level of a SASP marker, optionally measuring p53, p21, and/or p16, said determining whether said one or more compound(s) reduce(s) a level of at least one senescence-associated secretory phenotype protein comprises determining protein level(s) of one or more SASP markers, optionally determining p53, p21, and/or p16, e.g. using ELISA, Western blotting, and/or a functional assay such as a protease activity assay, and/or
said determining whether said one or more compound(s) reduce(s) a gene expression of at least one senescence-associated secretory phenotype gene comprises determining gene expression of one or more SASP markers, optionally determining *p53, p21,* and/or *p16*, e.g. using RT-PCR and/or mRNA half-life analysis, wherein, preferably, said SASP marker is selected from IL-1, IL-6, IL-8, IL-5, MIP-1α, and IFN-γ.

8. The method according to any one of the foregoing claims, wherein said method further comprises determining whether said one or more compound(s) reduce(s) a level of AU-rich mRNA, optionally a level of AU-rich mRNA of a senescence-associated secretory phenotype gene, in said treated sample compared to said control.

9. The method according to any one of the foregoing claims, wherein step a) comprises providing a sample comprising senescent cells, optionally a tissue sample, of a patient; and/or
wherein step a) comprises providing a sample comprising cells and inducing senescence in said cells using a senescence inducing agent, preferably a lipopolysaccharide, to provide a sample comprising senescent cells.

10. The method according to any one of the foregoing claims, wherein said sample comprises immune cells, preferably monocytes and/or macrophages; wherein, optionally, said sample comprises THP-1 cells.

11. A compound for use in a method of preventing or treating aging in a patient, preferably a patient having senescent cells or being at risk of acquiring senescent cells, wherein said senescent cells are **characterized by** an increased level of phosphorylated TTP in senescent cells compared to non-senescent cells;
wherein said compound is a compound identified using a method according to any one of claims 1-10, and/or
wherein said compound is selected from small molecule inhibitors, antibodies, and antigen-binding fragments thereof, preferably is selected from protein kinase inhibitors.

12. The compound for use according to claim 11, wherein said method of preventing or treating aging in a patient comprises the steps of:
i) providing a sample comprising cells, optionally a tissue sample, of said patient,
ii) determining the level of phosphorylated TTP in said sample,
iii) administering a therapeutically effective amount of said compound(s) to said patient, if there is an increased level of phosphorylated TTP in the sample compared to a control; wherein, preferably, said control is a reference value and/or is a level of phosphorylated TTP determined in a reference sample.

13. The compound for use according to claim 11 or 12, wherein said compound is selected from acetyldigitoxin, digitoxin, ergotamine, bromocriptine, dihydroergotoxine, dihydroergotamine, ergoloid mesylate, conivaptan, teniposide, tubocurarin, differin, dutasteride, itraconazole, paclitaxel, lurasidone, novobiocin, praziquantel, loperamide, pranlukast, fluspirilene, antrafenine, desloratadin, dihydroergotamine, ergotamine, glimepiride, glipizide, darifenacin, tadalafil, conivaptan, danazol, dutasteride, tasosartan, metocurine, nilotinib, telithromycin, irinotecan, 5-bromo-2-chloro-N-[3-methyl-4-(2-oxo-2H-chromen-3-yl)phenyl]benzamide, maraviroc, and saquinavir;
wherein, preferably, said compound is selected from glipizide, loperamide, praziquantel, glimepiride, fluspirilene, desloratadin, ergotamine, and teniposide.

14. The compound for use according to any one of claims 11-13, wherein said preventing or treating aging comprises preventing or treating immunosenescence and/or inflammaging; and/or
wherein said preventing or treating aging comprises preventing or treating an aging-associated disease, preferably selected from cardiovascular diseases such as atherosclerosis and artery narrowing e.g. artery stenosis, type-II diabetes, arthritis, Alzheimer's disease, Parkinson's disease, chronic obstructive pulmonary disease, cancer, and stroke.

15. A method of determining if a patient is likely to respond to a treatment with an anti-aging compound, preferably selected from small molecule inhibitors, antibodies, and antigen-binding fragments thereof, more preferably selected from protein kinase inhibitors, wherein the method comprises the following steps:
i) providing a sample of a patient, wherein said sample comprises senescent cells,
ii) determining a level of phosphorylated tristetraprolin (TTP) in said sample, and
iii) comparing the level of phosphorylated TTP determined in step ii) to a control, wherein said control is preferably a reference value and/or a reference sample,
wherein an increased level of phosphorylated TTP in said sample compared to said control indicates that said patient is likely to respond to a treatment using an anti-aging compound;
wherein, optionally, said method further comprises
providing a sample of said patient, and treating said sample with one or more anti-aging compound(s),
determining a level of phosphorylated TTP in said treated sample, and, comparing the level of phosphorylated TTP determined in said treated sample to the level of phosphorylated TTP determined in step ii),
wherein a decreased level of phosphorylated TTP in said treated sample compared to the level of phosphorylated TTP determined in step ii) indicates that said patient is likely to respond to a treatment with said one or more anti-aging compound(s).
